# EUROPEAN PATENT APPLICATION

(11) **EP 2 468 269 A1**
(43) Date of publication of application: **27.06.2012**
(21) Application number: 10192669.9
(22) Date of filing: 26.11.2010
(51) Int. Cl.: A61K 31/00, A61K 31/4245, A61K 31/454, A61K 31/5377, A61K 31/541, A61P 9/10

(54) **Nitric oxide donors in therapy of nitric oxide deficiency-induced disturbances of cerebral microcirculation**

(71) Applicant: Universitätsklinikum Münster, 48149 Münster (DE)
(72) Inventor: The designation of the inventor has not yet been filed
(74) Representative: Schiweck, Weinzierl & Koch

(57) **Abstract**

The present invention relates to nitric oxide releasing compounds and their use in the prevention, amelioration and/or therapy of nitric oxide (NO) deficiency induced disturbances of the cerebral microcirculation. The NO deficiency induced disturbances of the cerebral microcirculation may be due to an intracranial hemorrhage or stroke and can cause cerebrovascular spasms (or cerebral vasospasms) (CVS) and/or malperfusion of brain parenchyma caused by blood vessel and blood flow dysregulation which, in turn, can cause secondary neurological deficiencies (DIND) and/or brain infarction. Particularly, the present invention relates to the prevention, amelioration and/or therapy of delayed cerebral vasospasm-associated disorders after survived subarachnoidal hemorrhage by applying a NO-donor, most preferably Molsidomine.

## Description

### TECHNICAL FIELD OF THE INVENTION

The present invention relates to nitric oxide releasing compounds and their use in the prevention, amelioration and/or therapy of nitric oxide (NO) deficiency induced disturbances of the cerebral microcirculation. The NO deficiency induced disturbances of the cerebral microcirculation may be due to an intracranial hemorrhage or stroke and can cause cerebrovascular spasms (or cerebral vasospasms) (CVS) and/or malperfusion of brain parenchyma caused by blood vessel and/or blood flow dysregulation which, in turn, can cause secondary neurological deficiencies (DIND) and/or brain infarction. Particularly, the present invention relates to the prevention, amelioration and/or therapy of delayed cerebral vasospasm-associated disorders after survived subarachnoidal hemorrhage by applying a NO-donor, most preferably Molsidomine.

### BACKGROUND OF THE INVENTION

Brain hemorrhage (bleeding) is a type of stroke. It can, for example, be caused by an artery in the brain bursting and causing localized bleeding in the surrounding tissues. This bleeding kills brain cells. Brain hemorrhages are also called cerebral hemorrhages, intracranial hemorrhages, or intracerebral hemorrhages. They account for about 13% of strokes.
When blood from trauma irritates brain tissues, it causes swelling. This is known as cerebral edema. The pooled blood collects into a mass called a hematoma. These conditions increase pressure on nearby brain tissue, and that reduces vital blood flow and kills brain cells. Bleeding can occur inside the brain, between the brain and the membranes that cover it, between the layers of the brain's covering or between the skull and the covering of the brain.

A well-known phenomenon in medicine is an aneurism in the brain which, upon burst, causes a so-called subarachnoid hemorrhage (SAH). According to a review of 51 studies from 21 countries, the average incidence of subarachnoid hemorrhage is 9.1 per 100,000 annually. Studies from Japan and Finland show higher rates in those countries (22.7 and 19.7, respectively), for reasons that are not entirely understood. South and Central America, in contrast, have a rate of 4.2 per 100,000 on average (10, 72).
Despite obliterating the offending aneurysm and removing the risk of re-bleeding, up to half of the treated patients develop a syndrome of focal and/or cognitive deficits due to cerebral vasospasm (delayed ischemic neurological deficit, symptomatic vasospasm) between the fourth and ninth day after the SAH. As a result, many die or suffer permanent morbidity, and it has been described as the single most important cause of morbidity and mortality in patients whose ruptured aneurysm is successfully treated. Patients require vigilant monitoring and treatment for up to 2-4 weeks, including invasive monitoring of blood pressure, cerebral blood flow and metabolism and often complex treatment with calcium antagonists, hypertensive drugs, hemodilution and hypervolemia (triple H therapy), plus risky and often only temporarily effective intra-arterial administration of vasodilator drugs or balloon angioplasty.

Cerebral vasospasm after subarachnoid haemorrhage (SAH) is a vasoconstriction of the large intradural brain arteries forming the circle of Willis, developing with a delay of about four to 14 days after the bleeding. Vasospasm represents the most important reason for secondary neurological deficits after aneurismal SAH. Thus, patients who already suffer from the consequences of SAH are at a high risk of being burdened with vasospasm that may even cause brain infarction. At present 30 - 40% of the patients develop a remaining neurological deficit and symptomatic brain infarctions caused by cerebral vasospasm, 15-20% a high-grade lesion or die as a consequence thereof Due to advanced CT technique in up to 50% of patients CVS associated brain infarction can be detected(1, 2, 5, 6). Angiographically a vasospasm may be demonstrated at about 60 to 70% of the patients. Not in every case a clinical deterioration of the neurological findings is associated with an angiographically demonstrable vasospasm. In such situations, spasms may be assumed in the area of the micro-circulation (4, 8, 9). The occurrence and intensity of cerebral vasospasms correlate to the quantity of the coagulated blood attached to the vessels (1,3).Up to now, since 1989 no further therapeutic approach for the prophylaxis and therapy of spasm-associated neurological deficits after SAH has been proved sufficient by clinical studies (1, 3, 4).

Up to now, no dependable therapeutic approach for the prophylaxis and therapy of spasm-associated or otherwise generated delayed neurological deficits after brain hemorrhage, in particular SAH has been proved by well-designed clinical studies (1-4, 10). Only orally administered Nimodipine exerted a beneficial effect with regard to cerebrovascular spasm (CVS), DIND and poor outcome (3, 11). Under Nimodipine therapy, 20-30% spasm-associated infarcts (up to 50% detected by advanced CT-technique), 27-37% associated DINDs and a spasm-related mortality and high-grade morbidity of 30-40% are expected (1, 2, 5, 6, 7, 12, 13). As regards DIND, a good outcome is expected for 9% of the patients with DIND and a bad outcome (high-grade disability, coma, death) is expected for 62% (14). Only in small cohorts and case studies, some locally administered drug delivery compounds (Nicardipine or NO-donor-loaded pellets) seemed to prevent vasospasm and DIND and improve outcome without exerting severe side effects, however, a confirmation in a powerful randomized study is still missing (2, 15, 64-66, 84).

The most important vasodilatator of cerebral arteries is nitrogen monoxide (NO), produced in the healthy endothelium of vessels by (inter alia) constitutive synthases in a continuous basal manner (16-18,21). In addition, NO is produced by neurons, glia, endothelium and perivascular parasympathic nerve fibers, the latter mainly originate from sphenopalatine ganglion and vascular SMC (16, 19) either constitutively or after stimulation. Apart from endothelium-derived NO, astrocyte- and neuron-released NO contributes to the control of cerebral blood vessels function and cortical blood flow (16). In summary, relaxations of large cerebral arteries, resistance vessels and small cerebral arteries and, thus, consequently the control the cortical blood flow and supply, are strictly dependent on the presence of NO (16, 20, 21, 25). A lack of the basal NO-availability would cause a vasoconstriction due to elevated levels of endothelin and rho-kinase (21-23, 67-70) and due to the underlying basic myogenic tone of cerebral arteries (10). After, in particular SAH, a significant decrease up to total disappearance of endothelial NOS and related mRNA in great and small cerebral arteries is proven, leading to impaired vasodilatation (16, 24, 46, 62).
NO is not only involved in the regulation of the current tone of cerebral vessels, but moreover displays further biological effects - in part per expression of an inducible isoform of NOS (33). For instance, vascular preservation through proliferation inhibition of smooth muscle in the vessel wall and stimulation of repair processes in endothelial cells for control of quiescent state (34-41,58), an inhibition of the thrombocyte aggregation or leukocyte adhesion (18, 42) and an induction or depression of different enzymes and receptors, amongst other of plasmine, a suppression of PDGF from platelets (leads to cell proliferation and migration in smc, thus structural alteration of vessel wall), angiotensin-II receptor and the NMDA-receptor (17, 22, 23,28-32, 35, 42-45). Last, but not least, Nitric oxide is a potent free radical scavenger, who controls the local redox-equilibrium, and maintains a homeostasis in the endothelium for example on cellular level (19, 22, 26-32, 73, 81, 82)

It is assumed that hemoglobin and its degradation products (oxy-hemoglobin, metabolites of the hemoglobin) of the blood/blood clot attached to the cerebral vessels after brain hemorrhage, in particular after SAH, play a decisive, pluripotent role in spasm, without being bound by theory, mostly by counteracting NO together with destroying NOS and/or by generating free oxygen specimen, lipid peroxydation, inflammation, inducing synthesis of vasoconstrictors (46-48, see below) and destruction of vessel wall and mechanical subjecting cerebral vessels to pressure.
In order to treat the above mentioned pathophysiological conditions the intrathecal, local or intraventricular application of sodium nitroprusside (SNP), GTN or other NO donors have shown a good prophylactic effect on CVS and ischemia (most likely if given early after onset) in humans or laboratory animal (4, 6, 10, 49-56). But there are substantial undesired adverse characteristics (short half-value period, hypotension, "steal effect", increasing ICP, a nitrate tolerance and the cyanide-content, no permission for use in men), especially of SNP (3, 4, 53, 57). For avoidance of the undesired adverse effects, examinations using an other NO-donor have been carried out, but did not proof to be efficient and/or acceptable for a patient.

Summing up, delayed cerebrovascular spasms in micro- and macrocirculation following brain hemorrhage such as subarachnoid hemorrhage (SAH) (induced by an NO deficiency) are the most important reason for secondary neurological deficiencies (DIND) and brain infarction (in 20-40% of all patients). The risk for CVS correlates to the clot volume. The clot and its degradation products are considered as main reasons for spasm and probably in part DIND. They can bind and destroy the most important vasodilatator and second messenger Nitroxide (NO) directly and destruct the endothelium together with the NO-synthases leading to a serious endothelial dysfunction, destruct neuronal NOS and perivascular NOS which may cause an absolute or relative lack of NO (42, 46, 62, 85). They can induce serious and permanent histological changes in vascular wall (46). Reasons for DIND are suspected to be multifactorial, but mainly based on a lack of available NO in micro-environment (10, 62) and not solely depending on presence of CVS in macrocirculation (61).. However, up to now, prophylaxis as well as therapy has not proved satisfactory. Indeed, a multicenter study applying the selective endothelin 1A inhibitor Clazosetan, which showed to be highly efficiency for the treatment of CVS, for the treatment and/or prevention of CVS, but failed to show any effect on long-term outcome or clinical benefit (10, 14, 63, 83). There was a dose dependent trend towards lower spasm related brain infarction in a re-exploration in the CONCIOUS-1 (83) and a meta-analysis (63), but this did not reach statistical relevance.
Accordingly, there is a great demand and need to prevent, ameliorate and/or treat disturbances of the cerebral microcirculation induced by a nitric oxide (NO) deficiency. Indeed, NO deficiency induced disturbances can cause cerebrovascular spasms (CVS) which, in turn, can cause secondary, delayed ischemic neurological deficiencies (DIND) and/or brain infarction. Hence, research aims at developing new therapeutic approaches to benefit patients suffering from these unfavorable medical conditions such as delayed cerebral vasospasm-associated disorders after survived subarachnoidal hemorrhage. Accordingly, the technical problem underlying the present invention is to comply with the demand and need set out in the art and to thus provide means and methods for the prevention, amelioration and/or treatment of disorders associated with NO deficiency-induced disturbances of the cerebral macro- and microcirculation.

### SUMMARY OF THE INVENTION

The present invention provides as a solution to the technical problem means and methods for the prevention, amelioration and/or treatment of disorders associated with NO deficiency-induced disturbances of the cerebral microcirculation by applying a NO-donor, most preferably Molsidomine.

In fact, the present inventors have proven the pharmacotherapeutic effect of a NO donor such as Molsidomine in patients suffering from a NO deficiency-induced disturbance of the cerebral microcirculation.

A nitro-donor with a favourable pharmacodynamic and pharmacokinetic profile is molsidomine. The active component of molsidomine is SIN-1, which is hepatically separated from molsidomine and shows a half-live of 1-2 hours. In clinical dosages, SIN-1 does not show any toxicity, no relevant plasma protein binding and associated interactions, no accumulation, a linear dose-effect, a large therapeutic dose range, has been established in the treatment of coronary heart diseases does usually not lead to a relevant compromise of systematic haemodynamics or therapy limiting dose dependent hypotension, may be applied intravenously and does not show any tolerance development.
The main effects of SIN-1 consist in the relaxation of arterioles and large venous and arterial vessels independently from an intact endothelium, a decrease of the tone of the smooth vascular muscles, a concentration-depending inhibition of the platelet aggregation and release of platelet derived vasoactive compounds like thromboxane (equipotent to indometacine), an increase of the intracellular cyclic-GMP-concentration plus increased and sustained nitrite/nitrate levels and an inhibition of adenosine, thrombine, serotonin, collagen and "platelet activating factor". Thus far, Molsidomine is applied for the treatment of coronary spasms and it was not thought to be beneficial for the treatment and/or prevention of vasospasms in the brain when admimistered intravenously and/or orally.

In detail, the present inventors carried out a first treatment attempt on 27 patients (after approval of the respective authorities and under consent of the patients) having developed spasms, some of them under nimodipine treatment. These patients received Molsidomine i.v. as of the initiation of spasms (definition: mean flow velocity in the TCD>120 cm/sec, and after intra-arterial spasmolysis.

In a pilot trial 3/27 SAH patients were treated with Molsidomine compared to 49 patients that received standard therapy with Nimodipine alone. Thus, 25/49 patients were not treated with Molsidomine. The latter group developed vasospasm associated brain infarctions. As shown by follow up at least 3 months after discharge the present inventors found a formidable clinical benefit in patients receiving Molsidomine as measured by mNIH-SS and modified Rankin Scale (mRS) especially in patients with higher Hunt and Hess (H&H) grades (Molsidomine mean mNIH 4,58 and mRS 1,85 compared to Nimodipine alone treated persons mNIH 10,26 and mRS 3,83). Some patients treated with Molsidomine are partly in work process again or will be reintegrated soon (n=5 /27), the relatives are "very satisfied". Normally, approx. 5% of the patients return to their place of work. To the best of the present inventors's judgement, no serious, untreatable (i.e. severe hypotension) adverse effects were observed. There was a trend in Transcranial Doppeler (TCD)-values towards normal velocities, and some patients performed an on-off-effect.
In essence, treatment of SAH-survivors with Molsidomine showed 1.) no clinical deterioration, but 2.) a impressive improvement of clinical condition and long term outcome in the follow up and 3.) patients evolved substantially less vasospasm related brain infarctions at the rate of 1 : 4 in favour of Molsidomine patients.
It is thus considered that a significant improvement of cerebral microcirculation that is not detectable in TCD or DSA is the reason underlying the striking results obtained from the treatment of patients with severe SAH and CVS. In fact, the impact of microvessels in brain perfusion is 70% compared to 30% of bigger arteries (7-9, 10,16, 21, 62).
Hence, the favorable neurological outcome of patients suffering from brain hemorrhage, a decrease in CVS associated brain infarcts and a decrease in mortality when the NO-donor Molsidomine was applied make Molsidomine and, thus, a NO-donor an promising therapeutic substance in the treatment, amelioration and/or in particular prevention of NO-deficiency induced disturbances of the microcirculation, in particular, in the treatment and/or prevention of cerebrovascular spasms after SAH.
Additionally it should be borne in mind that mostly spasms of the brain's microcirculation seem to be the cause of brain infarcts, since, for example, the medicament Clazosentan or Nimodipine decrease spasms of the brain's macrocirculation, but do not necessarily improve a patient's neurological outcome, led the present inventors conclude that a NO-donor is beneficial for the treatment, amelioration and/or prevention of NO-deficiency induced disturbances of the cerebral microcirculation of a human subject. In fact, in the trials described herein patients with severe SAH and CVS that receive Molsidomine are subject to a surprising decrease of spasm related infarctions and a striking improvement of clinical outcome as measured by NIH-SS and mRS. Another interesting aspect of the present inventors' study is the fact that they found a good outcome without an outstanding Triple H therapy or hypertension. The MAP of 65 to 70 mmHG was kept in normal ranges.

Accordingly, the present inventors' finding is materialized in the following items of the present invention:
[1] A nitric oxide (NO) donor for use in the treatment, prevention and/or amelioration of nitric oxide deficiency-induced disturbances of the cerebral microcirculation of a human subject.
[2] The NO donor of item [1], which is a compound having the formula I 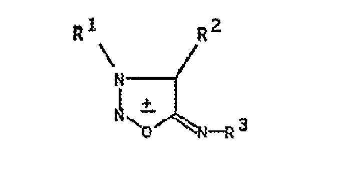 wherein
   R¹ is morpholine, N-heterocyclyl or N(R⁴)₂;
   R² is H, alkyl, halogen, alkenyl, alkynyl, aryl, aralkyl, aralkenyl, alkylene, alkenylene, cycloalkyl, cycloalkylene or N-heterocyclyl;
   R³ is H, -NO, -SO₂R⁴, -C(O)OR⁴, -C(O)R⁴, -CH₂NHC(O)R⁴, -CHR⁴, -NR⁴, -CHR⁴OC(O)R⁴ and -C(O)CHR⁴N⁺H₂; and
   each R⁴ is independently hydrogen, alkyl, alkenyl, alkynyl, aryl, aralkyl or aralkenyl;
   or a pharmaceutically acceptable salt of said compound.
[3] The NO donor of item [2], wherein R¹ is morpholine, R² is H and R³ is -C(O)OC₂H₅.
[4] The NO donor of any of items [1] to [3], which is N-ethoxycarbonyl-3-morpholinosydnonimine (molsidomine).
[5] The NO donor of item [4], which has the formula II 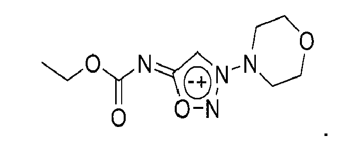
[6] The NO donor of item [2], wherein R¹ is morpholine, R² is H and R³ is H.
[7] The NO donor of item [5], which is 3-morpholinosydnonimine (SIN-1).
[8] The NO donor of item [7], which has the formula III 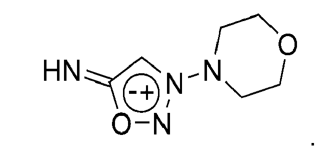
[9] The NO donor of item [2], which is 3-(3,3-dimethyl-1-oxo-1,4-thiazine-4-yl)sydnonimine, 3-(3,3-dimethyl-1,1-dioxo-1,4-thiazine-4-yl)sydnonimine, 3-(3,3-dimethyl-1,4-thiazine-4-yl)sydnonimine, 3-(cis-2,6-dimethylpiperidino)sydnonimine, 3-morpholinosydnoniminechloride (Linsidomine; SIN-1) or 3-(cis-2,6-dimethylpiperidino)-N-(4-methoxybenzoyl)sydnonimine (Pirsidomin) or N-ethoxycarbonyl-3-morpholinosydnonimine (Molsidomine).
[10] The NO donor of item [1], which is selected from the group consisting of L-arginine, L-citrulline, nitroglycerin (GTN), isosorbide 5-mononitrate (ISMN), isosorbide dinitrate (ISDN), pentaerythritol tetranitrate (PETN), erythrityl tetranitrate (ETN), amino acid derivatives such as N-hydroxy-L-arginine (NOHA), N.sup.6-(1-iminoethyl)lysine) (L-NIL), L-N.sup.5-(1-iminoethyl)ornithine (LN-NIO), and N.sup.a-methyl-L-arginine (L-NMMA), S-nitrosoglutathione (SNOG), S,S-dinitrosodithiol (SSDD), N-[2-(nitroxyethyl)]-3-pyridinecarboxamide (nicorandil), S-nitroso-N-acetylpenicillamine (SNAP), DEA-NONOate (2-(N,N-diethylamino)-diazenolate-2 - oxide), spermine NONOate (N-[4-[1-(3-aminopropyl)-2-hydroxy-2-nitrosohydrazino]butyl-1,3-propanediamine), 3-(5'-hydroxymethyl-2'-furyl)-1-benzyl indazole (YC-1), 8-bromo-cyclic-GMP (8-Br-cGMP), 8-(4-chlorophenylthio)guanosine-3',5'-cyclic monophosphate (8-PCPT-cGMP), sildenafil, cilostamide (N-cyclohexyl-N-methyl-4-(1,2-dihydro-2-oxo-6-quinolyloxy)butyramide, dipyridamole (2,6-bis(diethanol-amino)-4,8-dipipendinopyrimido-[5,4-d]pyrimidine), erythro-9-(2-hydroxy-3-nonyl)adenine (EHNA), etazolate (1-ethyl-4-[(1-methylethylidene)hydrazino]-1 H-pyrazolo-[3,4-b]-pyridine-5- carboxylic acid, ethyl ester), 4-{[3,4-(methylene-dioxy)benzyl]amino]}-6-chloroquinazoline (MBCQ), 8-methoxymethyl-1-methyl-3-(2-methylpropy3)xanthine (MMPX), 1-(3-chlorophenylammo)-4-phenyl-phthalazme (MY-5445), 4-(3-butoxy-4-methoxyphenyl)methyl-2-imidazolidone (Ro 20-1724), Rolipram (4-(3-(cyclopentyloxy)-4-methoxyphenyl)pyrrolidin-2-one), vinpocetine (3a,16a)-eburnamenine-14-carboxylic acid ethyl ester), zaprinast (2-propyloxyphenyl)-8-azapurin-6-one), and zardaverine (6-[4-(difluoro-methoxy)-3-methoxyphenyl]-3(2H)-pyridazinone).
[11] The NO donor of any one of the preceding items, wherein the NO donor is optionally further for use in the treatment, prevention and/or amelioration of NO deficiency-induced disturbances of the cerebral macrocirculation.
[12] The NO donor of any one of the preceding items, wherein the disturbances of the cerebral microcirculation and/or macrocirculation cause cerebrovascular spams (CVS) and/or malperfusion of brain parenchyma caused by blood vessel and blood flow dysregulation.
[13] The NO donor of any one of the preceding items, wherein the cerebrovascular spasms cause secondary neurological deficiencies (DIND) and/or brain infarction.
[14] The NO donor of any one of the preceding items, wherein the NO deficiency-induced disturbances are due to an intracranial hemorrhage or stroke.
[15] The NO donor of any one of the preceding items, wherein the intracranial hemorrhage results from a traumatic or a non-traumatic cause.
[16] The NO donor of any one of the preceding items, wherein intracranial hemorrhage is an intra-axial hemorrhage (cerebral hemorrhage) or extra-axial hemorrhage.
[17] The NO donor of any one of the preceding items, wherein the intra-axial hemorrhage is an intraparenchymal hemorrhage, intraventricular hemorrhage, or intraventricular traumatic diffuse bleeding.
[18] The NO donor of any one of the preceding items, wherein the intra-axial hemorrhage is caused by brain trauma, hemorrhagic stroke and/or spontaneous bleeding into the brain tissue.
[19] The NO donor of any one of the preceding items, wherein the extra-axial hemorrhage is extra-axial chronical response acute bleeding.
[20] The NO donor of any one of the preceding items, wherein the extra-axial hemorrhage is an epidural, subdural or subarachnoid hemorrhage.
[21] The NO donor of any one of the preceding items, wherein the subarachnoid hemorrhage is spontaneous or traumatic.
[22] The NO donor of any one of the preceding items, wherein the extra-axial hemorrhage is caused by an aneurysm or trauma.
[23] The NO donor of any one of the preceding items, wherein said NO donor is administered prior to, concurrently and/or after NO deficiency-induced disturbances.
[24] The NO donor of any one of the preceding items, wherein said NO donor is administered intravenously, intra-arterially and/or locally.
[25] The NO donor of any one of the preceding items, wherein said NO donor is administered continuously.
[26] The NO donor of any one of the preceding items, wherein said NO donor is administered at a dose such that the mean arterial pressure (MAP) is maintained at at least 65 mm Hg.
[27] The NO donor of any one of the preceding items, wherein said NO donor is administered at a dose of more than about 2 mg per hour.
[28] The NO donor of any one of the preceding items, wherein said NO donor is administered for a time sufficient to stabilize the clinical outcome of the human subject.
[29] The NO donor of any one of the preceding items, wherein said NO donor is administered for at least 7 days.
[30] The NO donor of any one of the preceding items, wherein said NO donor is administered orally after its intravenous, intra-arterial and/or local administration is terminated.
[31] A dosage regime for the treatment, amelioration and/or prevention of nitric oxide (NO) deficiency-induced disturbances of the cerebral microcirculation of a human subject comprising
   (a) continuously administering the NO donor as defined in any one of items [1] to [10] intravenously, intra-arterial and/or locally prior to, concurrently and/or after NO deficiency-induced disturbances of the cerebral microcirculation such that the mean arterial pressure (MAP) is maintained at at least 65 mm Hg; and
   (b) continuously administering the NO donor as defined in any one of items [1] to [10] orally after its intravenous, intra-arterial and/or local administration is terminated.
[32] The dosage regime of item [31], wherein the compound of (a) is administered at a dose of at least 2 mg per hour.
[33] The dosage regime of item [31] or [32], wherein the compound of (a) is administered for at least 7 days.
[34] The dosage regime of any one of items [31] to [33], wherein the compound of (b) is administered at a dose of about 2 mg once per day.
[35] The dosage regime of any one of items [31] to [34], wherein the compound of (b) is administered for at least 7 days.
[36] A kit comprising a NO donor as defined in any one of items [1] to [10] and instructions for administering said compound in accordance with the dosage regime of any one of items [31] to [35].
[37] A pharmaceutical package or kit comprising one or more intravenous doses and one or more oral doses as defined in any one of the preceding items.
[38] A compound as defined in any one of items [1] to [10] for the treatment, amelioration and/or prevention of nitric oxide (NO) deficiency-induced disturbances of the cerebral microcirculation of a human subject, wherein said compound is prepared to be administered in accordance with the dosage regime as defined in any one of items [31] to [35].

### DETAILED DESCRIPTION OF THE INVENTION

The inventors of the present application have proven the pharmacotherapeutic effect of a NO donor in patient suffering from a NO deficiency-induced disturbance of the cerebral microcirculation. It is contemplated that NO-related protective effects other than a directly vasodilatatory one are causative for the observation of a significant improvement of cerebral microcirculation. This improvement in cerebral microcirculation induced by any NO mediated reason is considered to be causative for the striking results obtained from the treatment of patients with severe SAH and CVS. This is supported by the finding that the vasospasm of small vessels of the microcirculation has more impact on the clinical outcome than the CVS of the larger blood vessels of the macrocirculation.

A NO donor according to the present invention is a promising therapeutic substance in therapy of NO deficiency-induced disturbances of the cerebral microcirculation. This may be due to a regulation of the NO misbalance-caused dysfunction of endothelium and endogenous production of NO. After administration of a NO donor according to the present invention in a patient suffering from a NO deficiency-induced disturbance of the cerebral microcirculation, a significant improvement of clinical outcome could be detected, and a significantly lower rate of cerebrovascular infarctions and mortality was observed. Specifically, a NO donor according to the present invention, most preferably Molsidomine, is a promising therapeutic substance in the prevention and/or treatment of CVS after SAH, with prevention being most preferred.

The present invention provides the use of a nitric oxide (NO) donor (including an NO releasing substance) in the manufacture of a medicament for the treatment, prevention and/or amelioration of NO deficiency-induced disturbances of the cerebral microcirculation of a human subject. Likewise, the present invention provides a NO-donor (preferably a composition comprising a NO-donor) for use in the treatment, prevention and/or amelioration of NO deficiency-induced disturbances of the cerebral microcirculation of a human subject. Additionally, the present invention provides a NO-donor (preferably a composition comprising a NO-donor) for use in a method of treatment, amelioration and/or prevention of NO deficiency-induced disturbances of the cerebral microcirculation of a human subject comprising administering a therapeutically effective amount of said composition.

"Prevention" includes that NO deficiency induced disturbances of the cerebral microcirculation may be avoided before they occur. For example, a NO donor may be applied to a patient prior to brain surgery or any other potential irritation of the brain surface that may cause an NO deficiency induced disturbace of the cerebral microcirculation as described herein.

Thus, in various embodiments the NO donor is used in the manufacture of a medicament for the treatment, prevention and/or amelioration of nitric oxide deficiency-induced disturbances of the cerebral microcirculation, wherein the NO donor is optionally further for use in the treatment, prevention and/or amelioration of NO deficiency-induced disturbances of the cerebral macrocirculation.

In various embodiments, the NO deficiency-induced disturbances of the cerebral microcirculation and/or macrocirculation cause cerebral vasospasms (can be equally used herein with the term "cerebrovascular spasms") (CVS) and/or malperfusion of brain parenchyma caused by blood vessel and/or blood flow dysregulation. In various embodiments, said cerebral vasospasms cause secondary neurological deficiencies (DIND) and/or brain infarction.

While intracerebral hemorrhage (ICH) is bleeding directly into the brain tissue, forming a gradually enlarging hematoma (pooling of blood), intracranial hemorrhage is the accumulation of blood anywhere within the skull vault. In the present invention, the NO deficiency-induced disturbances of the cerebral microcirculation and/or macrocirculation are preferably NO deficiency-induced disturbances of the cerebral microcirculation and/or macrocirculation due to an intracranial hemorrhage or stroke. An outstanding aspect is a therapeutic preconditioning and a resulting improved ischaemia tolerance of brain at risc by nitroxide (for example perioperatively). The result of such a pre-conditioning is a better cytoprotection, angio- and neuro-neogenesis (86). NO contributes to a limitation of infarction volumes and an improvement of the outcome after infarction. This is finally done based on an improvement of the CBF after preconditioning by NO, scavenging of free oxygen species, inhibition of caspase-3, the antiinflammatory and anti-thrombotic characteristics, but also a stimulation of growth factors and proteins such as VEGF and phosphoinositide 3-kinase, which stops the neuronal apoptosis at least in the penumbra. Nitroxide limits the damage due to cortical spreading (87-91), stops the transport of excitatory amino acids transporters through cell membranes (92) and inhibits the immunological response and activation of microglia triggered by ischemic events -phagocytosis and secretion of chemo- and cytokines by microglia (93,94). Accordingly, as mentioned above, the present invention also envisages that an NO donor is administered to a subject prior to any measure such as brain surgery that may cause nitric oxide deficiency-induced disturbances of the cerebral microcirculation of a human subject.

In various embodiments, said intracranial hemorrhage results from a traumatic or a non-traumatic cause.

A distinction is made between intra-axial hemorrhage (blood inside the brain) and extra-axial hemorrhage (blood inside the skull but outside the brain). Intra-axial hemorrhage is due to intraparenchymal hemorrhage or intra-ventricular hemorrhage (blood in the ventricular system). In the present invention, the NO deficiency-induced disturbances of the cerebral microcirculation and/or macrocirculation due to an intracranial hemorrhage are preferably NO deficiency-induced disturbances of the cerebral microcirculation and/or macrocirculation due to an intra-axial hemorrhage (cerebral hemorrhage) or an extra-axial hemorrhage.

Preferably, said intra-axial hemorrhage is an intraparenchymal hemorrhage, an intraventricular hemorrhage, or intraventricular traumatic diffuse bleeding. In various embodiments, said intra-axial hemorrhage and extra-axial hemorrhage result from a traumatic or a non-traumatic cause.

Likewise, in various embodiments said intraparenchymal hemorrhage, intraventricular hemorrhage, and intraventricular traumatic diffuse bleeding result from a traumatic or a non-traumatic cause.

In various embodiments, the intra-axial hemorrhage is caused by brain trauma, hemorrhagic stroke and/or spontaneous bleeding into the brain. Likewise, in various embodiments the intraparenchymal hemorrhage, intraventricular hemorrhage, or intraventricular traumatic diffuse bleeding is caused by brain trauma, hemorrhagic stroke and/or spontaneous bleeding into the brain.

The main types of extra-axial hemorrhage are epidural hematoma (bleeding between the dura mater and the skull), subdural hematoma (in the subdural space) and subarachnoid hemorrhage (SAH) (between the arachnoid mater and pia mater). In the present invention, the extra-axial hemorrhage is preferably extra-axial chronical response acute bleeding. In various embodiments, the extra-axial hemorrhage according to the present invention is an epidural, subdural or subarachnoid hemorrhage (SAH). Therefore, in various embodiments the NO deficiency-induced disturbances of the cerebral microcirculation and/or macrocirculation are preferably NO deficiency-induced disturbances of the cerebral microcirculation and/or macrocirculation due to an epidural hemorrhage, subdural hemorrhage (such as chronic subdural hemorrhage) or subarachnoid hemorrhage (SAH). Similarly, NO deficiency-induced disturbances of the cerebral microcirculation and/or macrocirculation are preferably NO deficiency-induced disturbances of the cerebral microcirculation and/or macrocirculation due to irritation of the brain surface due to, for example, surgical methods such as tumor resection, bypass operation or revascularization by myosangiosss at the brain or post-operative hemorrhage in the brain. More preferably, in the present invention the NO deficiency-induced disturbances of the cerebral microcirculation and/or macrocirculation are preferably NO deficiency-induced disturbances of the cerebral microcirculation and/or macrocirculation due to delayed cerebral vasospasm (DCV) and delayed ischemic neurological deficit (DIND) after SAH. For the latter preferred embodiment, Molsidomine is most preferably applied.

In various embodiments, the extra-axial hemorrhage according to the present invention results from a non-traumatic or traumatic cause. More preferably, the extra-axial hemorrhage according to the present invention is caused by an aneurysm or a head trauma (head injury).

Likewise, in various embodiments the epidural, subdural or subarachnoid hemorrhage according to the present invention results from a non-traumatic or traumatic cause. More preferably, in various embodiments the epidural, subdural or subarachnoid hemorrhage according to the present invention is caused by an aneurysm or a head trauma (head injury). In various embodiments, the extra-axial hemorrhage according to the present invention is spontaneous, preferably spontaneous due to an aneurysm. Likewise, in various embodiments the epidural, subdural or subarachnoid hemorrhage according to the present invention is spontaneous, preferably spontaneous due to an aneurysm.

In various embodiments, DCV after SAH according to the present invention results from a non-traumatic or a traumatic cause. More preferably, DCV after SAH according to the present invention is caused by an aneurysm or head trauma (head injury). In various embodiments, DCV after SAH according to the present invention is spontaneous, preferably spontaneous due to an aneurysm.
As described herein, in various embodiments intracranial hemorrhage is preferably an extra-axial hemorrhage. Preferably, the extra-axial hemorrhage is an extra-axial chronical response acute bleeding. In various embodiments, the extra-axial hemorrhage is preferably an epidural hemorrhage, a subdural hemorrhage or a subarachnoid hemorrhage. In various preferred embodiments, the extra-axial hemorrhage as described herein is caused by an aneurysm or trauma (head trauma).

In various embodiments, the NO donor is administered to a human subject in need thereof prior to, concurrently and/or after occurrence of NO deficiency-induced disturbances of the cerebral microcirculation and/or macrocirculation according to the present invention.

In various embodiments, the NO donor is administered to a human subject in need thereof continuously. More preferably, the NO donor is administered to a human subject in need thereof by continuous infusion, more preferably continuous intravenous infusion. Alternatively, the NO donor may be administered transdermally, intraparenchymatously, perivascularly or intraarterially. For these administration routes the NO donor may be in the form of gels, pellets or the like. In various embodiments, the NO donor is administered to a human subject in need thereof by continuous oral administration, *i.e*. administration of oral dosages subsequently over a period of time. In various embodiments, the NO donor is administered continuously according to the present invention at least until mean blood flows in the trans-cranial doppler (TCD) are normalized. Alternatively, the NO donor is administered as long as CVS is suspected to occur and/or as long as nimodipine is administered. The person skilled in the art is in the position to determine and monitor normalization of mean blood flows in the TCD in the treatment, prevention and/or amelioration of a human subject according to the present invention.

In various embodiments, the NO donor is administered to a human subject in need thereof over a period of at least four (4) days, preferably over a period of at least five (5) days, more preferably over a period of at least six (6) days. Still more preferably, the NO donor is administered to a human subject in need thereof for at least seven (7) days, even more preferably for at least eight (8) days. In various embodiments, the NO donor is administered to a human subject in need thereof for at least nine (9) days, preferably for at least ten (10) days. The administration over the indicated period of days is particularly beneficial to prevent NO-induced disturbances of the cerebral microcirculation, in particular when Molsidomine is administered.
In various embodiments, the NO donor is administered to a human subject in need thereof at a dose such that the mean arterial pressure (MAP) is maintained at at least 65 mm Hg, preferably at at least 70 mm Hg, more preferably at at least 75 mm Hg. Still more preferably, the NO donor is administered to a human subject in need thereof at a dose such that the MAP is maintained at at least 80 mm Hg, even more preferably at at least 85 mm Hg or at least 90 mm Hg.

Advantageously, molsidomine as a particularly preferred NO donor of the present invention may be initially administered at a dose of 0.5-1mg/h with a slight increase of its amount over time. However, the blood pressure has to be controlled so that it does not lead to hypotension. Simultaneously, catecholamines can be administered so that molsidomine can be administered at a dose of preferably 3.5 - 10 mg/h. Accordingly, arterenol (a catecholamine) may be administered at a dose of 0.1 - 0.2 µg/h.
In various embodiments, the NO donor is administered to a human subject in need thereof at a dose of at least/more than about 2 mg/h, preferably at a dose of at least/more than about 3 mg/h. In various embodiments, the NO donor is administered to a human subject in need thereof at a dose between about 3.5 to 10 mg/h, preferably at a dose between about 4.5 to 9 mg/h. More preferably, the NO donor is administered to a human subject in need thereof at a dose between about 5.5 to 8 mg/h, still more preferably at a dose between about 6.5 to 7 mg/h.
In various embodiments, the NO donor according to the present invention is administered to a human subject in need thereof at a dose of about 2 mg once per day, preferably about 2.5 mg once per day, more preferably about 3 mg once per day, even more preferably at a dose of about 3.5 mg once per day. For example, Molsidomine as NO-donor may be administered in accordance with the scheme(s) described in the appended Examples.

In various embodiments, the NO donor is administered to a human subject in need thereof perorally (orally), intravenously, intra-arterially and/or locally, wherein locally means locally to the site of the disturbance of the cerebral microcirculation and/or macrocirculation according to the present invention. In other words, the NO donor may be administered by surgical implantation at the diseased site.

Preferably, the NO donor is administered to a human subject in need thereof by intrathecal administration. Intrathecal administration is the introduction of a therapeutic substance into the cerebrospinal fluid by injection into the subarachnoid space of the spinal cord or intraventriculary in order to bypass the blood-brain barrier.

In various embodiments, the NO donor is administered to a human subject in need thereof perorally (orally) after its intravenous, intra-arterial, and/or local administration in accordance with the present invention has been terminated. That is, in the present invention it is contemplated that the route of administration of the NO donor is changed during the treatment, prevention and/or amelioration of a NO deficiency-induced disturbance of the cerebral microcirculation and/or macrocirculation of a human subject. In various embodiments where the NO donor according to the present invention is orally administered to a human subject in need thereof after its intravenous, intrathecal, intra-arterial, and/or local administration is terminated, the NO donor is administered orally to the human subject in need thereof at a dose of about 2 mg once per day, preferably about 2.5 mg once per day, more preferably about 3 mg once per day, even more preferably at a dose of about 3.5 mg once per day.

The present invention also provides a dosage regime for the treatment, amelioration and/or prevention of NO deficiency-induced disturbances of the cerebral microcirculation and/macrocirculation of a human subject comprising (a) continuously administering a NO donor according to the present invention intravenously, intra-arterially and/or locally prior to, concurrently and/or after occurrence of NO deficiency-induced disturbances of the cerebral microcirculation and/or macrocirculation such that the mean arterial pressure (MAP) is maintained at at least 65 mm Hg, and (b) continuously administering the NO donor according to the present invention orally after its intravenous, intra-arterial and/or local administration is terminated.

Furthermore, the present invention provides a kit or container comprising a NO donor or compound according to the present invention and instructions for administering the NO donor or compound in accordance with the dosage regime according to the present invention. Accordingly, the kit or container holds a therapeutically effective amount of a pharmaceutical composition of NO donor and instructions for using the pharmaceutical composition for treating, preventing or ameliorating a pathological condition in accordance with the present invention.

Still further, the present invention provides a pharmaceutical package or kit comprising one or more intravenous doses and/or one or more oral doses of a NO donor in accordance with the dosage regime according to the present invention.

Also provided by the present invention is a composition comprising a NO donor according to the present invention for the treatment, amelioration and/or prevention of NO deficiency-induced disturbances of the cerebral microcirculation and/macrocirculation of a human subject, wherein said composition is prepared to be administered in accordance with a dosage regime according to the present invention.
Likewise, the present invention also provides a compound as described herein for the treatment, amelioration and/or prevention of NO deficiency-induced disturbances of the cerebral microcirculation and/macrocirculation of a human subject, wherein said compound is prepared to be administered in accordance with a dosage regime according to the present invention.

As described herein, the NO donor, active compound or composition used in the present invention may be administered in combination with other composition(s) and/or drug(s), as long as such other composition(s) and/or drug(s) are applicable, *i.e*. as long as the do not adversely affect the treatment, prevention and/or amelioration of NO deficiency-induced disturbances according to the present invention. A combined administration as described herein may be in the form of a simultaneous or sequential administration of the NO donor, active compound or composition according to the present invention and the other composition(s) and/or drug(s).

Examples of other compositions and/or drugs include Nimodipine and/or Clazosentan.
In the present invention, nitric oxide donor is intended to mean any compound which mimics the effects of NO, generates or releases NO through biotransformation, any compound which generates NO spontaneously, any compound which spontaneously releases NO, or any compound which in any other manner generates NO or a NO-like moiety when administered to a mammal. Such a compound can also be referred to as a "NO mimic", "NO prodrug", "NO producing agent", "NO delivering compound", "NO generating agent", "NO provider", or "compound containing NO". In the present invention, said terms may be used interchangeably. In the present invention, an NO donor donates nitric oxide or a related redox species and more generally provides nitric oxide bioactivity, that is activity which is identified with nitric oxide, e.g., vaso-relaxation or stimulation or inhibition of a receptor protein, *e.g.*, ras protein, adrenergic receptor, NMB. NO donors including S-nitroso, O-nitroso, C-nitroso and N-nitroso compounds and nitro derivatives thereof and metal NO complexes, but not excluding other NO bioactivity generating compounds, useful herein, are described in "Methods in Nitric Oxide Research", edited by Feelisch, M., and Starnler, J.S., John Wiley & Sons, New York, 1996, pages 71-115, which is incorporated herein by reference. NO donors which are C-nitroso compounds where nitroso is attached to a tertiary carbon and which are useful herein include those described in U.S. Patent Application No. 09/695,934 which has matured into U. S. Patent No. 6,359, 182 and those described in WO 02/34705.

The most preferred NO donors useful in the present invention are molsidomine and SIN-1, respectively. While molsidomine is described herein as N-ethoxycarbonyl-3-morpholinosydnonimine, *N*-(Ethoxycarbonyl)-3-(4-morpholinyl)-sydnonimine is a term found in the literature that likewise describes the molsidomine useful in the present invention.

After oral administration, molsidomine is completely absorbed and undergoes an enzymatic transformation (hydrolysis and decarboxylation) in the liver. The SIN-1 produced is itself rapidly transformed in the blood, without enzymatic intervention, to SIN-1A. SIN-1 and SIN-1A are the active metabolites of molsidomine. SIN-1A is then degraded by oxidation to inactive SIN-1C with the release of NO. SIN-1C is then itself metabolized in the liver, as described in the document by Bernd Rosenkranz et al., Clinical Pharmacokinetics of Molsidomine, Clin. Pharmacokinet. 1996, May; 30 (5) 372-384. Molsidomine is currently marketed in Europe under the trademark name Corvaton®. Molsidomine generally acts for a period of 4 to 5 hours for a 4 mg dose and 10 to 12 hours for an 8 mg dose.
Molsidomine is on the market in the form of tablets containing 2, 4 or 8 mg of the active ingredient. It may be advantageous from the point of view of the comfort of the patient to have galenical forms of the medicament presenting a longer therapeutic effect. This would allow reducing the number of daily intakes of the drug. Therefore, in the context of present invention, delayed-release formulations of molsidomine, which are for instance described in WO-A1-01/62256, may be administered in accordance with the present invention. For example, WO-A1-01/62256 discloses an oral galenical form of molsidomine with delayed-release, which contains an therapeutic quantity of molsidomine or one of its active metabolites and which shows an in vitro dissolution rate of 15 to 25% of molsidomine release after 1 hour, of 20 to 35 % release after 2 hours, of 50 to 65 % release after 6 hours, of 75 to 95 % release after 12 hours, of more then 85 % release after 18 hours and of more then 95 % release after 24 hours; whereby the in vivo peak in plasmatic molsidomine comprising a concentration of 25 to 40 ng per ml plasma, is preferably reached within 3 to 4 hours after administration. The use of these molsidomine formulations is hereby incorporated by reference.

In various embodiments of the invention, a nitric oxide donor may be administered to the patient by a drug delivery system in minimal doses or microdoses, so as to provide dosages which are about one half to about one twentieth (1/2 to 1/20) of those known to induce vasodilation in healthy vasculature or non-symptomatic locations of the patient. The low doses of a NO donor effectively enhance NO and alleviate disturbances of cerebral microcirculation without inducing undesirable side effects such as systemic vasodilatation or headaches.

As described herein, microcirculation is the flow of blood cells and blood plasma in the smallest blood vessels having diameters of less than 200 µm. Microcirculation is functionally the most important part of blood circulation because here exchange of substances with the cells of the tissue is realized. This applies to the transportation of oxygen and substrates to the cells as wells as the transportation of metabolic end products away from the cells and the tissue. The specific functional state of microcirculation determines the required regulation with respect to an adaptation of microperfusion towards changing metabolic needs. Furthermore, undisturbed microcirculation is the prerequisite for non-restricted processing of the first steps of an immunological reaction. Here, the local regulation of microcirculation, specifically the autoregulation of brain resistance vessels and auto-rhythmic contraction of vascular smooth muscle cells are of particular importance.
Amongst others, important criteria for characterizing normal or impaired microcirculation are:
- the particular distribution state of the blood in the microvessel network;
- auto-rhythmic (spontaneous) vessel agitation in arterioles and venules (vasomotion);
- flow in arteriolar inflow and venular flow-off;
- rheologic features (local hematocrit);
- flow velocity of blood cells;
- diameter of micro-vessels.
The vaso-motoric functional state determines essentially the width of adjustment of microcirculation to changing metabolic needs and, therefore, the local width of regulation of microcirculation.
The person skilled in the art is in the position to obtain measurement data of blood microcirculation, said measurement data comprising features like the exhaustion of oxygen in the venules, blood flow in the venules, local hematocrit in the micro-vessels, spontaneous arteriolar vasomotion, state of vasomotion in the venules, and local changes in concentration of substances in tissue. A further explanation of these features is given at pages 5 and 6 of international patent application WO 2008/025731 A1, which describes non-invasive measuring methods on a target tissue in order to describe the aforementioned features of the microcirculation of the blood, while otherwise mainly relating to a device for generating a pulsed electromagnetic field with pulse control for improving microcirculation of the blood. The said explanations of the above-mentioned features given in WO 2008/025731 is specifically referred to herewith and, therefore, the corresponding disclosure of WO 2008/025731 A1 forms part of the present application. Furthermore, WO 2008/025731 A1 also includes at pages 6/7 a reference to the literature with respect to the basics for measurement of these features. By way of reference, this disclosure of WO 2008/025731 also forms part of the present application herewith.

In the present invention, the human subject suffering from a NO deficiency-induced disturbance of the cerebral microcirculation and/or macrocirculation according to the present invention may also be considered as a patient suffering from a NO deficiency-induced disturbance of the cerebral microcirculation and/or macrocirculation according to the present invention. In various embodiments, a patient in need of a treatment with a NO donor according to the present invention may be a patient that had received a treatment for treating, preventing and/or ameliorating a NO deficiency-induced disturbance of the cerebral macrocirculation prior to a treatment, prevention or amelioration of a NO deficiency-induced disturbance of the cerebral microcirculation according to the present invention.
In various embodiments, the human subject in need of a NO donor according to the present invention is tested prior to administration of the NO donor in order to determine the extent of impairment of the NO deficiency-induced cerebral microcirculation. Cerebral microcirculation and macrocirculation can be visualized by the skilled person using standard means available in the art, for example, orthogonal polarization spectral (OPS) imaging. The microvascular network may be visualized using an SDF videomicroscopy system (MicroScan^{™}, MicroVisionMedical Inc., Amsterdam, Netherlands) or a blood flow measuring probe or metabolic measuring probe or the like.

In various embodiments, the human subject according to the present invention is a human subject that has survived an intracranial hemorrhage or stroke according to the present invention. Preferably, said human subject that has survived an intracranial hemorrhage or stroke according to the present invention is a human subject that has survived a delayed cerebral vasospasm (DCV) after an intracranial hemorrhage or stroke according to the present invention. More preferably, said human subject that has survived a DCV according to the present invention is a human subject that has survived a DCV after a SAH according to the present invention. In various embodiments, the human subject in need of a NO donor according to the present invention is a human subject that has survived a brain infarction and/or a secondary neurological deficiency (DIND) according to the present invention. Preferably, the human subject in need of a NO donor according to the present invention is a human subject that has survived a brain infarction and/or a DIND post a DCV, wherein the DCV preferably is a DCV after an intracranial hemorrhage or stroke according to the present invention, and wherein said intracranial hemorrhage preferably is a SAH.

As described herein, the human subject in need of administration of a NO donor according to the present invention (*i.e*., a "human subject in need thereof", as described herein) is a human subject suffering from a NO deficiency-induced disturbance of the cerebral microcirculation and/or macrocirculation in accordance with the present invention. In other words, the "human subject in need thereof" is a human subject suffering from a NO deficiency-induced disturbance of the cerebral microcirculation and/or macrocirculation as described herein, and said subject is thus in need of a NO donor in accordance with the present invention, *i.e*. a NO donor useful in the treatment, prevention and/or amelioration of NO deficiency-induced disturbances of the cerebral microcirculation and/or macrocirculation in a human subject.
In NO deficiency-induced disturbances of the cerebral microcirculation, there is a primary tissue impairment followed by a secondary impairment of the affected tissue, which is due to inflammation occurring after a human subject had suffered a NO deficiency-induced disturbances of the cerebral microcirculation. In various embodiments, the NO donor according to the present invention provides for an inhibition of said secondary inflammation-based impairment of the affected tissue. As the secondary inflammation-based impairment of the tissue affected by a NO deficiency-induced disturbance of the cerebral microcirculation may be the cause of secondary neurological deficiencies (DIND) according to the present invention, a NO donor according to the present invention is useful in the treatment, prevention and/or amelioration of such DINDs according to the present invention. As the DINDs according to the present invention may be causative for morbidity and/or mortality of a human subject suffering from a NO deficiency-induced disturbance of the cerebral microcirculation, a NO donor according to the present invention is useful for the prevention of morbidity and/or mortality of a human subject suffering from a NO deficiency-induced disturbance of the cerebral microcirculation.

In accordance with the above, in one aspect of the present invention, the NO donor is administered to a human subject in need thereof for a time sufficient to stabilize the clinical outcome of the human subject suffering from a NO deficiency-induced disturbance of the cerebral microcirculation.
Accordingly, in one aspect, the present invention provides the use of a NO donor in the manufacture of a medicament for stabilizing and/or improving the clinical outcome of a human subject suffering from a NO deficiency-induced disturbance of the cerebral microcirculation in accordance with the present invention.
More specifically, the present invention provides the use of a NO donor in the manufacture of a medicament for the prevention of morbidity and/or mortality of a human subject, wherein the morbidity and/or mortality is related to a NO deficiency-induced disturbance of the cerebral microcirculation in accordance with the present invention. In other words, the present invention provides the use of a NO donor in the manufacture of a medicament for the prevention of morbidity and/or mortality of a human subject that had suffered a NO deficiency-induced disturbance of the cerebral microcirculation according to the present invention.

In various embodiments, said human subject that had suffered a NO deficiency-induced disturbance of the cerebral microcirculation is a human subject that may have been treated otherwise before being treated with a NO donor in order to prevent the morbidity and/or mortality that is related to the NO deficiency-induced disturbance of the cerebral microcirculation. That is, a human subject that had suffered a NO deficiency-induced disturbance of the cerebral microcirculation according to the present invention may have received a first treatment directed to treating a NO deficiency-induced disturbance of the cerebral macrocirculation. However, as said first treatment may not prevent the human subject's morbidity and/or mortality that is related to the NO deficiency-induced disturbance of the cerebral microcirculation, the human subject may receive a second treatment with a NO donor according to the present invention in order to prevent the human subject's morbidity and/or mortality that is related to the NO deficiency-induced disturbance of the cerebral microcirculation.

In various embodiments, said first and second treatment may be carried out simultaneously or sequentially, the latter meaning that said second treatment with a NO donor according to the present invention follows said first treatment. Thus, the present invention provides the use of a NO donor in the manufacture of a medicament for the prevention of a human subject's morbidity and/or mortality related to a NO deficiency-induced disturbance of the cerebral microcirculation post treatment of a NO deficiency-induced disturbance of the cerebral macrocirculation of the human subject.

As described herein, a NO donor to be used in the present invention is preferably at least one of L-arginine, L-citrulline, nitroglycerin (GTN), isosorbide 5-mononitrate (ISMN), isosorbide dinitrate (ISDN), pentaerythritol tetranitrate (PETN), erythrityl tetranitrate (ETN), amino acid derivatives such as N-hydroxy-L-arginine (NOHA), N.sup.6-(1-iminoethyl)lysine) (L-NIL), L-N.sup.5-(1-iminoethyl)ornithine (LN-NIO), and N.sup.a-methyl-L-arginine (L-NMMA), S-nitrosoglutathione (SNOG), S,S-dinitrosodithiol (SSDD), N-[2-(nitroxyethyl)]-3-pyridinecarboxamide (nicorandil), S-nitroso-N-acetylpenicillamine (SNAP), DEA-NONOate (2-(N,N-diethylamino)-diazenolate-2 - oxide), spermine NONOate (N-[4-[1-(3-aminopropyl)-2-hydroxy-2-nitrosohydrazino]butyl-1,3-propanediamine), 3-(5'-hydroxymethyl-2'-furyl)-1-benzyl indazole (YC-1), 8-bromo-cyclic-GMP (8-Br-cGMP), 8-(4-chlorophenylthio)guanosine-3',5'-cyclic monophosphate (8-PCPT-cGMP), sildenafil, cilostamide (N-cyclohexyl-N-methyl-4-(1,2-dihydro-2-oxo-6-quinolyloxy)butyramide, dipyridamole (2,6-bis(diethanol-amino)-4,8-dipipendinopyrimido-[5,4-d]pyrimidine), erythro-9-(2-hydroxy-3-nonyl)adenine (EHNA), etazolate (1-ethyl-4-[(1-methylethylidene)hydrazino]-1 H-pyrazolo-[3,4-b]-pyridine-5- carboxylic acid, ethyl ester), 4-{[3,4-(methylene-dioxy)benzyl]amino]}-6-chloroquinazoline (MBCQ), 8-methoxymethyl-1-methyl-3-(2-methylpropy3)xanthine (MMPX), 1-(3-chlorophenylammo)-4-phenyl-phthalazme (MY-5445), 4-(3-butoxy-4-methoxyphenyl)methyl-2-imidazolidone (Ro 20-1724), Rolipram (4-(3-(cyclopentyloxy)-4-methoxyphenyl)pyrrolidin-2-one), vinpocetine (3a,16a)-eburnamenine-14-carboxylic acid ethyl ester), zaprinast (2-propyloxyphenyl)-8-azapurin-6-one), and zardaverine (6-[4-(difluoro-methoxy)-3-methoxyphenyl]-3(2H)-pyridazinone).

More preferably, a NO donor according to the present invention is a compound having the following formula I or a pharmaceutically acceptable salt of said compound: 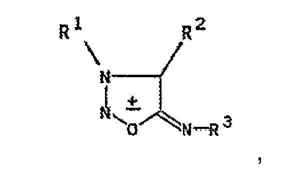 wherein R¹ is morpholine, N-heterocyclyl or N(R⁴)₂, R² is H, alkyl, halogen, alkenyl, alkynyl, aryl, aralkyl, aralkenyl, alkylene, alkenylene, cycloalkyl, cycloalkylene or N-heterocyclyl, R³ is H, -NO, -SO₂R⁴, -C(O)OR⁴, -C(O)R⁴, -CH₂NHC(O)R⁴, -CHR⁴, -NR⁴, -CHR⁴OC(O)R⁴ and - C(O)CHR⁴N⁺H₂, and each R⁴ is independently hydrogen, alkyl, alkenyl, alkynyl, aryl, aralkyl or aralkenyl. Preferably, in the compound according to the above formula I R¹ is morpholine, R² is H and R³ is -C(O)OC₂H₅.

Still more preferably, the NO donor to be used in the present invention is N-ethoxycarbonyl-3-morpholinosydnonimine (molsidomine). Molsidomine to be used in the present invention is marketed in Germany under the trademark name Corvaton®, and has the following formula II

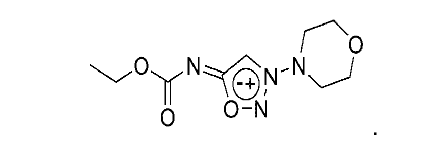

A preferred form of molsidomine to be used in the present invention is 3-morpholinosydnonimine (SIN-1). SIN-1 has the following formula III

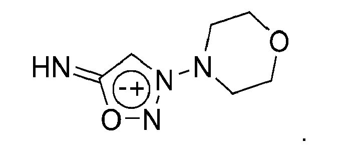

Accordingly, SIN-1 is a preferred NO donor to be used in the present invention.
In other preferred embodiments, in the compound according to the above formula I R¹ is morpholine, R² is H and R³ is H.

In still further preferred embodiments, the NO donor according to formula I is at least one of 3-(3,3-dimethyl-1-oxo-1,4-thiazine-4-yl)sydnonimine, 3-(3,3-dimethyl-1,1-dioxo-1,4-thiazine-4-yl)sydnonimine, 3-(3,3-dimethyl-1,4-thiazine-4-yl)sydnonimine, 3-(cis-2,6-dimethylpiperidino)sydnonimine, 3-morpholinosydnoniminechloride (Linsidomine; SIN-1), 3-(cis-2,6-dimethylpiperidino)-N-(4-methoxybenzoyl)sydnonimine (Pirsidomin) and N-ethoxycarbonyl-3-morpholinosydnonimine (Molsidomine).

As used in the specification and appended claims the following terms have the meaning indicated:
Each R⁵ is independently hydrogen, alkyl or aralkyl. Each R⁶ is straight or branched alkene chain optionally substituted by hydroxy, mercapto, alkylthio, aryl, cycloalkyl, -N(R⁴)₂, -C(O)OR⁴ or - C(O)N(R⁴)₂. Each R⁷ is independently hydrogen, alkyl or aralkyl.

"Alkyl" refers to a straight or branched hydrocarbon chain radical consisting solely of carbon and hydrogen atoms, containing no unsaturation, having from one to eight carbon atoms, and which is attached to the rest of the molecule by a single bond, e.g., methyl, ethyl, n-propyl, 1-methylethyl (iso-propyl), n-butyl, n-pentyl, 1,1-dimethylethyl (t-butyl), and the like. Unless stated otherwise specifically in the specification, the alkyl radical may be optionally substituted by hydroxy, alkoxy, aryloxy, haloalkoxy, cyano, nitro, mercapto, alkylthio, cycloalkyl, -N(R⁴)₂, - C(O)OR⁴, -C(O)N(R⁴)₂ or -N(R⁴)-C(O)-R⁴ where each R⁴ is as defined in the Summary of the Invention. Unless stated otherwise specifically in the specification, it is understood that for radicals, as defined below, that contain a substituted alkyl group that the substitution can occur on any carbon of the alkyl group.

"Alkoxy" refers to a radical of the formula -ORₐ where Rₐ is an alkyl radical as defined above, e.g., methoxy, ethoxy, n-propoxy, 1-methylethoxy (iso-propoxy), n-butoxy, n-pentoxy, 1,1-dimethylethoxy (t-butoxy), and the like. Unless stated otherwise specifically in the specification, it is understood that for radicals, as defined below, that contain a substituted alkoxy group that the substitution can occur on any carbon of the alkoxy group. The alkyl radical in the alkoxy radical may be optionally substituted as described above.
"Alkylthio" refers to a radical of the formula -SRₐ where Rₐ is an alkyl radical as defined above, e.g., methylthio, ethylthio, n-propylthio, 1-methylethylthio (iso-propylthio), n-butylthio, n-pentylthio, 1,1-dimethylethylthio (t-butylthio), and the like. Unless stated otherwise specifically in the specification, it is understood that for radicals, as defined below, that contain a substituted alkylthio group that the substitution can occur on any carbon of the alkylthio group. The alkyl radical in the alkylthio radical may be optionally substituted as described above.

"Alkenyl" refers to a straight or branched hydrocarbon chain radical consisting solely of carbon and hydrogen atoms, containing at least one double bond, having from two to eight carbon atoms, and which is attached to the rest of the molecule by a single bond or a double bond, e.g., ethenyl, prop-1-enyl, but-1-enyl, pent-1-enyl, penta-1 ,4-dienyl, and the like. Unless stated otherwise specifically in the specification, the alkenyl radical may be optionally substituted by hydroxy, alkoxy, haloalkoxy, cyano, nitro, mercapto, alkylthio, cycloalkyl, -N(R⁴)₂, -C(O)OR⁴, - C(O)N(R⁴)₂ or -N(R⁴)-C(O)-R⁴ where each R⁴ is as defined in the Summary of the Invention. Unless stated otherwise specifically in the specification, it is understood that for radicals, as defined below, that contain a substituted alkenyl group that the substitution can occur on any carbon of the alkenyl group.

"Alkynyl" refers to a straight or branched monovalent hydrocarbon chain radical consisting solely of carbon and hydrogen atoms, containing at least one triple bond, having from two to eight carbon atoms, and which is attached to the rest of the molecule by a single bond, e.g., ethynyl, prop-1-ynyl, but-1-ynyl, pent-1-ynyl, pent-3-ynyl, and the like. Unless stated otherwise specifically in the specification, the alkynyl radical may be optionally substituted by hydroxy, alkoxy, haloalkoxy, cyano, nitro, mercapto, alkylthio, cycloalkyl, -N(R⁴)₂, -C(O)OR⁴, -C(O)N(R⁴)₂ or -N(R⁴)-C(O)-R⁴ where each R⁴ is as defined in the Summary of the Invention. Unless stated otherwise specifically in the specification, it is understood that for radicals, as defined below, that contain a substituted alkynyl group that the substitution can occur on any carbon of the alkynyl group.

"Aryl" refers to a phenyl or naphthyl radical. Unless stated otherwise specifically in the specification, the term "aryl" or the prefix "ar-" (such as in "aralkyl") is meant to include aryl radicals optionally substituted by one or more substituents selected from the group consisting of alkyl, halo, nitro, cyano, haloalkyl, haloalkoxy, mercapto, alkylthio, phenyl, cycloalkyl, -OR⁴ (including hydroxy and alkoxy), -N(R⁴)₂, -R⁶-N(R⁴)₂, -N(R⁴)-C(O)OR⁷, -R⁶-N(R⁴)-C(O)OR⁷, - N(R⁴)-C(O)-R⁴, -R⁶-N(R⁴)-C(O)-R⁴, -C(O)OR⁴, -R⁶-C(O)OR⁴, -C(O)-N(R⁴)_{2,} -R⁶-C(O)-N(R⁴)₂, - C(O)-R⁶-N(R⁴)_{2,} -N(R⁵)-C(NR⁵)-N(R⁵)₂, -N(R⁵)-C(O)-N(R⁴)₂ and -N(R⁵)-C(O)-R⁶-N(R⁴)₂ where each R⁴, R⁵, and R⁶ are as defined above in the Summary of the Invention.

"Aralkyl" refers to a radical of the formula -RₐR_{b} where Rₐ is an alkyl radical as defined above and R_{b} is one or more aryl radicals as defined above, e.g., benzyl, diphenylmethyl and the like. The aryl radical(s) may be optionally substituted as described above.

"Aralkoxy" refers to a radical of the formula -OR_{d} where R_{d} is an aralkyl radical as defined above, e.g., benzyloxy, and the like. The aryl radical may be optionally substituted as described above.

"Aralkenyl" refers to a radical of the formula -R_{c}R_{b} where R_{c} is an alkenyl radical as defined above and R is one or more aryl radicals as defined above, e.g., 3-phenylprop-1-enyl, and the like. The aryl radical(s) and the alkenyl radical may be optionally substituted as described above.

"Alkylene chain" refers to a straight or branched divalent hydrocarbon chain consisting solely of carbon and hydrogen, containing no unsaturation and having from one to eight carbon atoms, e.g., methylene, ethylene, propylene, n-butylene, and the like. The alkylene chain may be optionally substituted by one or more substituents selected from the group consisting of aryl, halo, hydroxy, alkoxy, haloalkoxy, cyano, nitro, mercapto, alkylthio, cycloalkyl, -N(R⁴)₂, - C(O)OR⁴, -C(O)N(R⁴)₂ or -N(R⁴)-C(O)-R⁴ where each R⁴ is as described above in the Summary of the Invention. The alkylene chain may be attached to the rest of the molecule through any two carbons within the chain.

"Alkenylene chain" refers to a straight or branched divalent hydrocarbon chain consisting solely of carbon and hydrogen, containing at least one double bond and having from two to eight carbon atoms, e.g., ethenylene, prop-1-enylene, but-1-enylene, pent-1-enylene, hexa-1,4-dienylene, and the like. The alkenylene chain may be optionally substituted by one or more substituents selected from the group consisting of aryl, halo, hydroxy, alkoxy, haloalkoxy, cyano, nitro, mercapto, alkylthio, cycloalkyl, -N(R⁴)₂, -C(O)OR⁴, -C(O)N(R⁴)₂ or -N(R⁴)-C(O)-R⁴ where each R⁴ is as described above in the Summary of the Invention. The alkenylene chain may be attached to the rest of the molecule through any two carbons within the chain.

"Cycloalkyl" refers to a stable monovalent monocyclic or bicyclic hydrocarbon radical consisting solely of carbon and hydrogen atoms, having from three to ten carbon atoms, and which is saturated and attached to the rest of the molecule by a single bond, e.g., cyclopropyl, cyclobutyl, cyclopentyl, cyclohexyl, decalinyl and the like. Unless otherwise stated specifically in the specification, the term "cycloalkyl" is meant to include cycloalkyl radicals which are optionally substituted by one or more substituents independently selected from the group consisting of alkyl, aryl, aralkyl, halo, haloalkyl, hydroxy, alkoxy, haloalkoxy, cyano, nitro, mercapto, alkylthio, cycloalkyl, -N(R⁴)₂, -C(O)OR⁴, -C(O)N(R⁴)₂ or -N(R⁴)-C(O)-R⁴ where each R⁴ is as defined in the Summary of the Invention.

"Cycloalkylene" refers to a stable divalent monocyclic or bicyclic hydrocarbon consisting solely of carbon and hydrogen atoms, having from three to ten carbon atoms, and which is saturated and attached to the rest of the molecule by two single bonds, e.g., cyclopropylene, cyclobutylene, cyclopentylene, cyclohexylene, decalinylene and the like. Unless otherwise stated specifically in the specification, the term "cycloalkylene" is meant to include cycloalkylene moieties which are optionally substituted by one or more substituents independently selected from the group consisting of alkyl, aryl, aralkyl, halo, haloalkyl, hydroxy, alkoxy, haloalkoxy, cyano, nitro, mercapto, alkylthio, cycloalkyl, -N(R⁴)_{2,} -C(O)OR⁴, -C(O)N(R⁴)₂ or -N(R⁴)-C(O)-R⁴ where each R⁴ is as defined in the Summary of the Invention.

"N-heterocyclyl" refers to a stable 3- to 15-membered ring radical which consists of carbon atoms and from one to five heteroatoms selected from the group consisting of nitrogen, oxygen and sulfur wherein at least one of the heteroatoms is a nitrogen. For the purposes of this invention, the N-heterocyclyl radical may be a monocyclic, bicyclic or a tricyclic ring system, which may include fused or bridged ring systems; and the nitrogen, carbon or sulfur atoms in the N-heterocyclyl radical may be optionally oxidized; the nitrogen atom may be optionally quaternized; and the N-heterocyclyl radical may be partially or fully saturated or aromatic. The N-heterocyclyl radical may be attached to the main structure at any heteroatom or carbon atom which results in the creation of a stable compound. Examples of such N-heterocyclyl radicals include, but are not limited to, azepinyl, azetidinyl, benzimidazolyl, benzoxazolyl, carbazolyl, decahydroisoquinolyl, quinuclidinyl, imidazolyl, imidazolinyl, imidazolidinyl, isothiazolidinyl, indolyl, isoindolyl, indolinyl, isoindolinyl, indolizinyl, isoxazolyl, isoxazolidinyl, morpholinyl, benzothiadiazolyl, oxadiazolyl, octahydroindolyl, octahydroisoindolyl, 2-oxopiperazinyl, 2-oxopiperidinyl, 2-oxopyrrolidinyl, 2-oxoazepinyl, oxazolyl, oxazolidinyl, perhydroazepinyl, piperidinyl, piperazinyl, 4-piperidonyl, phenazinyl, phenothiazinyl, phenoxazinyl, phthalazinyl, pteridinyl, purinyl, pyrrolyl, pyrrolidinyl, pyrazolyl, pyrazolidinyl, pyridinyl, pyrazinyl, pyrimidinyl, pyridazinyl, quinazolinyl, quinoxalinyl, quinolinyl, quinuclidinyl, isoquinolinyl, thiazolyl, thiazolidinyl, thiadiazolyl, triazolyl, tetrazolyl, tetrahydroisoquinolyl, thiomorpholinyl, thiomorpholinyl sulfoxide, and thiomorpholinyl sulfone. The carbon atoms in the N-heterocyclyl radical may be optionally substituted by alkyl, halo, nitro, cyano, haloalkyl, haloalkoxy, mercapto, alkylthio, phenyl, cycloalkyl, -OR⁴, -N(R⁴)₂, -R⁶-N(R⁴)₂, -N(R⁴)-C(O)OR⁷, -R⁶-N(R⁴)-C(O)OR⁷, - N(R⁴)-C(O)-R⁴, -R⁶-N(R⁴)-C(O)-R⁴, -C(O)OR⁴, -R⁶-C(O)OR⁴, -C(O)-N(R⁴)₂, -R⁶-C(O)-N(R⁴)₂, - C(O)-R⁶-N(R⁴)₂, -N(R⁵)-C(NR⁵)-N(R⁵)₂, -N(R⁵)-C(O)-N(R⁴)₂ and -N(R⁵)-C(O)-R⁶-N(R⁴)₂ where each R⁴, R⁵, R⁶ and R⁷ are as defined above in the Summary of the Invention. The nitrogen atoms in the N-heterocyclyl may be optionally substituted by -C(NR⁵)-N(R⁵)₂, -C(NR⁵)-R⁴, -C(O)-N(R⁴)₂ or -C(O)-R⁶-N(R⁴)₂ where each R⁴, R⁵ and R⁶ and R⁷ are as defined above.

Preferred N-heterocyclyl radicals are piperidinyl, tetrahydrosoquinolinyl, or benzothiadiazolyl. "Halo" refers to bromo, chloro, fluoro or iodo.

"Haloalkyl" refers to an alkyl radical, as defined above, that is substituted by one or more halo radicals, as defined above, e.g., trifluoromethyl, difluoromethyl, trichloromethyl, 2,2,2-trifluoroethyl, 1-fluoromethyl-2-fluoroethyl, 3-bromo-2-fluoropropyl, 1-bromomethyl-2-bromoethyl, and the like.

"Haloalkoxy" refers to a radical of the formula -OR_{c} where R_{c} is an haloalkyl radical as defined above, e.g., trifluoromethoxy, difluoromethoxy, trichloromethoxy, 2,2,2-trifluoroethoxy, 1-fluoromethyl-2-fluoroethoxy, 3-bromo-2-fluoropropoxy, 1-bromomethyl-2-bromoethoxy, and the like.

As described herein, a NO deficiency-induced disturbance of the cerebral microcirculation and/or macrocirculation is a NO deficiency-induced disturbance of the cerebral microcirculation and/or macrocirculation according to the present invention, i.e. a NO deficiency-induced disturbance of the cerebral microcirculation and/or macrocirculation as described herein.
In the present invention, the term NO donor will be used to denote both the free form and the salified form of a NO donor according to the present invention.
The NO donors, active compounds and compositions used in the present invention include classic pharmaceutical preparations. Administration of the NO donors, active compounds and compositions according to the present invention will be via any common route as long as the target tissue is available via that route. In the present invention, the terms "medicament" and "pharmaceutical composition" may be used interchangeably.

The pharmaceutical forms suitable for injectable use include sterile aqueous solutions or dispersions and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi. The carrier can be a solvent or dispersion medium containing, for example, water, ethanol, polyol (for example, glycerol, propylene glycol, and liquid polyethylene glycol, and the like), suitable mixtures thereof, and vegetable oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents (for example, sugars or sodium chloride). Prolonged absorption of the injectable compositions can be brought about by the use in the compositions of agents delaying absorption (for example, aluminum monostearate and gelatin). Sterile injectable solutions are prepared by incorporating the active compounds in the required amount in the appropriate solvent with several of the other ingredients enumerated above, as required, followed by filtered sterilization. Generally, dispersions are prepared by incorporating the various sterilized active ingredients into a sterile vehicle that contains the basic dispersion medium and the required other ingredients from those enumerated above. In the case of sterile powders for the preparation of sterile injectable solutions, the preferred methods of preparation are vacuum-drying and freeze-drying techniques that yield a powder of the active ingredient plus any additional desired ingredient from a previously sterile-filtered solution thereof.
The NO donors, active compounds and compositions used in the present invention may be formulated in a neutral or salt form. Within the framework of the present invention, the NO donor can be used in free form, but also in the form of a pharmaceutically acceptable salt.
The therapeutic compounds may also be formulated for sustained release, for example, using microencapsulation; see WO 94/07529, and U.S. Patent No. 4,962,091, the disclosure of which pertaining to microencapsulation hereby forms part of the disclosure of the present application. The pharmaceutical compositions or formulations used in the present invention may include, as optional ingredients, pharmaceutically acceptable carriers, diluents, solubilizing or emulsifying agents, and salts of the type that are available in the art. Examples of such substances include normal saline solutions such as physiologically buffered saline solutions and water. Specific nonlimiting examples of the carriers and/or diluents that are useful in the pharmaceutical compositions and formulations of the present disclosure include water and physiologically acceptable buffered saline solutions, such as phosphate buffered saline solutions. Merely by way of example, the buffered solution can be at a pH of about 6.0-8.5, for instance about 6.5-8.5, or about 7-8.
The NO donor can be adjusted to the appropriate concentration, and optionally combined with other agents. The absolute weight of a given NO donor included in a unit dose can vary. The pharmaceutically acceptable carriers useful in this disclosure are conventional. See, e.g., Remington's Pharmaceutical Sciences, by E. W. Martin, Mack Publishing Co., Easton, PA, 15th Edition (1975). In general, the nature of the carrier will depend on the particular mode of administration being employed. For example, for solid compositions (e.g., powder, pill, tablet, or capsule forms), conventional non-toxic solid carriers can include, for example, pharmaceutical grades of mannitol, lactose, starch, or magnesium stearate. In addition to biologically-neutral carriers, pharmaceutical compositions to be administered can contain minor amounts of non-toxic auxiliary substances, such as wetting or emulsifying agents, preservatives, and pH buffering agents and the like, for example sodium acetate or sorbitan monolaurate.
Upon formulation, solutions used in the present invention will be administered in a manner compatible with the dosage formulation and in such amount as is therapeutically effective. The formulations are easily administered in a variety of dosage forms such as injectable solutions, drug release capsules and the like. For administration in an aqueous solution, for example, the solution should be suitably buffered if necessary and the liquid diluent first rendered isotonic with sufficient saline or glucose. Generally, effective amounts of a NO donor used in the present invention will be determined by the age, weight, and condition or severity of disease of the recipient. See Remington's Pharmaceutical Sciences, by E. W. Martin, Mack Publishing Co., Easton, PA, 15th Edition (1975), pages 697-773, herein incorporated by reference. Further to the definitions given elsewhere herein, those of ordinary skill in the art will readily optimize effective dosages and administration regimens as determined by good medical practice and the clinical condition of the individual patient.
In the present invention, the frequency of dosing may depend on the pharmacokinetic parameters of the compounds used in the present invention and the routes of administration. The optimal pharmaceutical formulation will be determined by one of skill in the art depending on the route of administration and the desired dosage. See, for example, Remington's Pharmaceutical Sciences, supra, pages 1435-1712, incorporated herein by reference. Such formulations may influence the physical state, stability, rate of in vivo release and rate of in vivo clearance of the administered compounds according to the present invention. Further refinement of the calculations necessary to determine the appropriate treatment dose is routinely made by those of ordinary skill in the art without undue experimentation, especially in light of the dosage information and assays disclosed herein, as well as the pharmacokinetic data observed in animal or human clinical trials.
The NO donors, active compounds and compositions used in the present invention are administered in therapeutically effective amounts, which means that a quantity is used sufficient to achieve a desired effect in a patient being treated in accordance with the present invention. The NO donors according to the present invention may also be delivered via an ultrasonic delivery system.

As described herein, "preferred embodiment" means "preferred embodiment of the present invention". Likewise, as described herein, "various embodiments" and "another embodiment" means "various embodiments of the present invention" and "another embodiment of the present invention", respectively.

In the present invention, the terms "cerebral vasospasm" and "cerebrovascular spasm" are used interchangeably. Thus, herein "CVS" is an abbreviation for both said terms.

As used in the specification and appended claims the following terms have the meaning indicated: epidural means between the dura mater (the outermost meninx) and the skull; subdural means in the subdural space between the dura and the arachnoid mater; subarachnoid means between the arachnoid and pia meningeal layers.

In various embodiments of the present invention, the term "treatment, prevention and/or amelioration" is to be read as "at least one of treatment, prevention and a melioration".

The National Institutes of Health stroke scale (NIH-SS) is a graded neurological examination that assesses speech, language cognition, inattention, visual field abnormalities, motor and sensory impairments, and ataxia. The scale was developed for use in acute-stroke therapy trials and has since been widely used as a standard part of the assessment in clinical trials. This scale, along with many others, has been evaluated in its clinical usefulness in the assessment of the stroke patient. The NIH-SS permits a high-resolution evaluation of a patient's neurological status. To obtain the finding on the NIH-SS, various neurological aspects are investigated and assigned point scores. The total point score is a measure of the severity of the symptoms of stroke, the point score increasing with the severity of the symptoms. This rating scale is also suitable for monitoring the course of the symptoms after stroke and for quantifying the success of any treatment used. In general, it is possible to establish a correlation between the size of the infarct and the severity of the stroke as quantified by the stroke scale. Hence, the course of the size of the infarct under treatment is also suitable for the assessment of a treatment effect.
Rating scales such as the NIH-SS or the modified Rankin scale are generally used for the quantitative evaluation of the severity of a cerebral disorder, whether acute or under treatment.
TCD (trans-cranial doppler) is a diagnostic procedure using ultrasound waves to measure blood flow through the major blood vessels of the brain. In particular, the purpose of this test is to detect any narrowing or blockage in arteries located at the base of the brain that may decrease or stop the flow of blood to the brain and increase blood flow velocities according to narrowed vessel diameter. The test is most useful at detecting decreased blood flow through narrow areas inside blood vessels. TCDs are especially useful for monitoring blood flow in the brain of stroke patients who are affected by brain swelling and vasospasm (which are cerebral blood vessel spasms).

### EXAMPLES

A better understanding of the present invention and of its advantages is given from the following examples, which are offered for illustrative purposes only and which are not intended to limit the scope of the present invention in any way.

The pharmacotherapeutic effect of molsidomine in delayed vasospasm associated DIND and brain infarctions as well as outcome in patients with SAH after aneurysmal rupture was proven. 3/27 patients being treated with molsidomine and 25/49 patients not being treated with molsidomine developed vasospasm associated brain infarctions. As shown by follow up at least three months after discharge the inventors found a formidable clinical benefit measured by mNIH-SS and modified Rankin Scale (mRS), specifically in patients with higher Hunt and Hess (H&H) grades (mean mNIH 4.58 and mean mRS 1.85 in molsidomine-treated patient group compared to mNIH 10.26 and mean mRS 3.83 in Nimodipine-treated patient group). No adverse side effects were observed using molsidomine. There was a trend in TCD-values towards normal velocities, and some patients performed a dramatically on-off-effect. Treatment of SAH-survivors with molsidomine showed (1.) no clinical deterioration, but (2.) an impressive improvement of clinical condition and long term outcome in the follow up, and (3.) patients evolved substantially less vasospasm related brain infarctions at a rate of 1:4 in favor of molsidomine-treated patients.
As for therapy failures under continuous nimodipin therapy and after having shown CVS in the TCD (mean flow >120 cm/sec) or angiographically, molsidomine in highest possible dosage maintaining a MAP >65 mmHg was used additionally to standard therapy in a therapeutic attempt using if necessary moderate catecholamine (Arterenol 0,1 - 0,2 µg/h) therapy under standard ICU conditions. If severe hypotension occurs, there might be an interruption of Molsidomin infusion for maximal 48h.
27 SAH patients were treated with Molsidomine and were compared with 49 SAH patients in the ICU under standard Nimodipine therapy with or without CVS. CCT at the beginning and after treatment were analyzed independently by two experienced neuroradiologists and were scanned for development of delayed brain infarction. At least three months after discharge a modified NIH-SS and a modified Rankin Scale (mRS) for clinical follow up was assessed.

### Patients

83 patients admitted to the Intensive Care Unit of the University Hospital of Münster, Germany, after sponateus aneurysmal SAH were registered in an observational study. Seven patients died in the first 124 hours after admission, without detected CVS probably related to malignant brain edema and severity of initial SAH. These patients were excluded from further evaluation.
All patients received standard ICU treatment, conditions and procedures, treated according to the standard therapy protocol with Nimodipine 5-10 ml/h i.v. or 6 x 60 mg p.o.. Nimodipine is a dihydropyridine derivative with the name 1,4-dihydro- 2,6-dimethyl-4-(3-nitrophenyl)-3,5-pyridinedicarboxylic acid 2-methoxyethyl 1- methylethyl ester. Nimodipine is a calcium channel antagonist and is the most rigorously studied and only drug approved by the US Food and Drug Administration (FDA) for use in the treatment of vasospasm (71).
Due to evidence for CVS (TCD, clinical) all patients received diagnostic DSA and a rescue therapy with local, intra-arterial Nimodipine infusions over 20 minutes. The treatment with molsidomine was applied if vasospasm was detected by TCD (mean flow >120mm/sec resp. Lindegaard-Index >3) or DSA (narrowing of vessel diameter >50%).
Out of 76 individuals 27 patients received molsidomine i.v. in an individually up-titrated dosage with a target quantity of 3.5-10 mg/h dependent on systemic circulation reactions. The remaining 49 patients received standard therapy.
Within 24h after admission to the ICU patients were treated with coil embolization and/or clipping. In the molsidomine group 22 patients were treated with coil embolization and 8 patients were treated with clipping, 3 patients could not be sufficiently coiled and therefore clipping was performed afterwards (= both treatments) . In the standard therapy group the relation was 32 (Coil), 16 (Clip), 1 both and 2 no aneurysm closure respectively Inclusion criteria for patients were: Appearance of CVS. Male and female patients with an age of 18 years and written informed consent for the additional application of molsidomine. Spontaneous SAH after aneurysmal rupture and a clinical stage of Hunt and Hess grade I to V.
Exclusion criteria for patients were: No detected CVS. Patients with SAH due to other pathological conditions, SAH without CT occurrence of blood, hypotension and low output below middle arterial pressure (MAP) of 65 mm Hg in spite of application of Arterenol® with max. 0.1-0.2 µg/h lung edema, HOCM (hypertrophic obstructive cardiomyopathy) pregnancy, generalized cerebral edema or (expected) death in 96 hours.
The grade of Hunt and Hess was distributed equally between both groups, there was no difference in Fisher grading. See Table 1 below.

According to natural incidence, we found a difference in sex (male:female = 1:2) and a higher risc for CVS in younger people. So, the average age was significantly less in the molsidomine group, namely 44.4 years (distribution: 27-77 years), compared to 56.9 years (distribution: 17-86 years) in the standard therapy group (p = 0,046).

**Table 1: Distribution of Fisher-grading and Hunt&Hess-grading in the treatment groups**

| **Fisher-grade** | **Molsidomine** | **Nimodipine** |
|---|---|---|
| 1 | n=1 | n=3 |
| 2 | n=2 | n=3 |
| 3 | n=4 | n=13 |
| 4 | n=20 | n=30 |
| Total | n=27 | n=49 |

| **Hunt & Hess-grade** | **Molsidomine** | **Nimodipine** |
|---|---|---|
| I° | n=5 | n=5 |
| II° | n=1 | n=4 |
| III° | n=8 | n=12 |
| IV° | n=10 | n=25 |
| V° | n=3 | n=3 |
| Total | n=27 | n=49 |

Distribution of Fisher and Hunt and Hess Scores for the molsidomine and the Nimodipine group. p = 0.59 (Hunt & Hess), p = 0.69 (Fisher).

### Molsidomine treament scheme:

In both groups blood pressure, intracranial pressure (ICP) and saturation measurement of blood oxygen were observed continuously. TCD was performed daily or on demand with investigation of the internal carotid artery (ACI), medial cerebral artery (MCA), anterior cerebral artery (ACA) and basilar artery (BA). For monitoring the clinical condition the GCS score (Glasgow Coma Score) was determined per hour
The drug used contains molsidomine, as supplied by Sanofi-Aventis, Germany, in ampullae with 2mg/molsidomine (Corvaton®). The dosage of molsidomine was adapted to the systemic blood pressure. The desired molsidomine dosage was in the range of 3.5-10mg/h and therefore distinctly higher than the dosage applied for the treatment of coronary vasospasm (being 0.6-1 mg/h).
The dosage was reduced if the MAP dropped below 65 mmHg despite infusion therapy (HES 6-10%) or a mild Arterenol® therapy (max dosage Arterenol® 0.1-0.2 µg/h). With increased need for Arterenol® the dosage of molsidomine was reduced accordingly. Patients with cardiac low-output failure induced by preexistent cardiac failure, stop of perfusion during acute SAH itself or other reasons like sepsis received molsidomine up-titrated in highest possible amount based on cardiac treatment or the treatment was interrupted for maximal 48 hours if Arterenol®- dosage escalated.
The treatment with molsidimine was continued (either i.v. or subsequent orally 4 x 8 mg ret.) until mean flows in TCD were normalized. The duration of molsodomine application was at least 8 days according to inclusion criteria: Appearance or presence of detectable CVS.
CCT and DSA images were independently reported by two experienced neuroradiologists for infarction and vasospasm. CVS related infarcts were diagnosed if hypodense lesions could be detected 72h or later after therapeutic intervention or operation in comparison to the 24h control CCT scan and being accompanied by clinical deterioration due to CVS or positive Doppler ultrasound or CVS in DSA.
After a minimum of three months the modified NIH and modified Rankin status were determined in order to control clinical outcome of patients (NIH-SS and mRS).

### Results

In all cases molsidomine did not show any relevant side effects. Even with higher dosage the measurement of MAP revealed only slight depressions of 10-20 mmHg (systolic) and 10 mmHg maximum in MAP. The MAP was consequently kept above 65-70 mmHg.
ICP did not increase for short or longer periods. Blood stains were normal. Fast reduction of molsidomine dosage caused an on/off phenomena or a rebound respectively, which was regredient after increased molsidomine levels.
As shown in Table 2 below, the occurrence of spasmus related infarcts was distinctly higher in the Nimodipine group for higher Hunt and Hess scores (p = 0,0005023). In the Nimodipine group 25 patients developed CVS (51%) associated infarcts, compared to 3 out of 27 in the molsidomine group (11.1%). One patient suffered from infarction ten days after stop of molsidomine treatment.

**Table 2: Spasmus related infarcts**

| **Hunt & Hess grade** | **Infarction Nimodipine group** | **Infarction Molsidomine group** |
|---|---|---|
| I° | 4 | 1 |
| II° | 1 | 0 |
| III° | 4 | 1 |
| IV° | 14 | 1 |
| V° | 2 | 0 |

### Outcome in relation to initial Hunt & Hess grading

Subdivided for initial Hunt & Hess grades, in particular for higher Hunt & Hess grades a striking improvement for Molsidomine treated patients was found (see Table 2). Differences in NIH-SS between Nimodipine and molsidomine groups were highly significant (p = 0.017), but did not reflect cases of death. Therefore, we used mRS and detected an outstanding discrepancy (p = 0.0004) comprising fatalities.

### Outcome in Nimodipine group

As shown in Table 3, the mean NIH was 10.26 and the mean mRS was 3.69 for the Nimodipine group, especially the patients presenting with Hunt and Hess III-V showed worse clinical conditions.

**Table 3: Outcome in Nimodipine group**

| **Hunt & Hess grade** | **Nimodipine group** | **Mean NIH** >3 months (without deaths) | **Mean mRS** >3 months |
|---|---|---|---|
| I° | n=5 | 0.5 | 1.6 |
| II° | n=4 | 0 | 3 |
| III° | n=12 | 10.6 | 3.75 |
| IV° | n=25 | 12.3 | 4.3 |
| V° | n=3 | 38 (2 x exitus) | 5.7 |
| Total | n=49 | Mean: 10.26 | Mean: 3.69 |

### Outcome in Molsidomine group

As shown in Table 4, for the molsidomine group mean NIH was 4.58 and mean mRS was 1.85. Especially the patients presenting with Hunt & Hess score III-V were in better clinical condition than in the Nimodipine group (Table 3).

**Table 4: Outcome in molsidomine group**

| **Hunt & Hess grade** | **Molsidomine group** | **Mean NIH** >3 months (without deaths) | **Mean mRS** >3 months |
|---|---|---|---|
| I° | n=5 | 0 | 1,75 |
| II° | n=1 | 1 | 1 |
| III° | n=8 | 3,62 | 1,75 |
| IV° | n= 10 | 3,8 | 1,6 |
| V° | n =3 | 17 | 4 |
| Total | n=27 | Mean: 4.58 | Mean: 1.85 |

### Deaths within three months post SAH

As shown in Table 5, the molsidomine group presents with a distinctly lower mortality rate (p = 0.0137) than the Nimodipine group.

**Table 5: Deaths within three months post SAH**

| **Hunt & Hess grade** | deaths <124h excluded without spasm | Nimodipine group (3 months) | molsidomine group (3 months) |
|---|---|---|---|
| I° | | n=1 | n=1 |
| II° | | n=2 | n=0 |
| III° | | n=2 | N=0 |
| IV° | | n=7 | n=0 |
| V° | n=7 | n=2 | n=0 |
| Total | | n=14 | n=1 |

### CVS frequency detected by TCD

Keeping in mind our inclusion criteria, we registered a highly significant discrepancy regarding frequency of detected CVS by TCD, indicating a critical condition of Molsidomine group members (p = 0,000056). Mean numbers of supraselective intra-arterial Nimodipine treatment per individual was equal (p = 0.41), indicating a weak effect of Molsidomine in terminating CVS. There was no difference in presence of CVS in DSA (p = 0,09).

**Table 6: CVS frequency detected by TCD**

| **Nimodipine group** | **CVS yes** | **CVS no** |
|---|---|---|
| | n=28 | n=18 |
| | 60.9% | 39.1% |

| **Molsidomine group** | **CVS yes** | **CVS no** |
|---|---|---|
| | n=27 | n=0 |
| | **100**% | 0% |

The delayed cerebral vasospasm in micro- and macrocirculation is probably the most important reason for delayed neurological deficits (DIND) and brain infarction in patients after subarachnoid hemorrhage (SAH). The blood clot and its degradation products are regarded as causative for vasospasm and associated disorders and at least in part for DIND. The risk for CVS correlates to the clot volume. Hitherto, preventive as well as symptomatic therapies have not been proven to be satisfactory. Molsidomine as a well known NO-donor is commonly used for coronary spasms (59, 60). In the present invention, molsidomine is used for the first time in the context of CVS after SAH for prophylaxis of DIND and CVS related cerebral infarctions.

### LITERATURE

1. Dorsch 2002: Therapeutic approaches to vasospasm in subarachnoid hemorrhage. Curr. Opin. Crit. Care 8(2):128-33.
2. Biondi et al. 2004 Intra-arterial Nimodipine for the treatment of symptomatic cerebral vasospasm after aneurysmal subarachnoid hemorrhage: Preliminary results. AJNR Am. J. Neuroradiol. 25:1067-76.
3. Pickard et al. 1989: Effect of oral Nimodipine on cerebral infarction and outcome after subarachnoid haemorrhage: British aneurysm Nimodipine trial. BMJ. 298:636-42.
4. Woertgen et al. 2003: Comparison of the Claassen and Fisher CT classification scale to predict ischemia after aneurysmatic SAH. Zentralbl. Neurochir. 64(3): 104-8.
5. Dorsch 1995: Cerebral arterial spasm - a clinical review. Br. J. Neurosurg. 9:403-12.
6. Corsten et al. 2001: Contemporary management of subarachnoid hemorrhage and vasospasm: the UIC experience. Surg. Neurol. 56(3):140-8.
7. Mocco et al. 2006: A review of current and future medical therapies for cerebral vasospasm following aneurismal subarachnoid hemorrhage. Neurosurg. Focus 21 (3):E9.
8. Vergouwen et al. 2008: Microthrombosis after aneurysmal subarachnoid hemorrhage: an additional explanation for delayed cerebral ischemia. J. Cereb. Blood Flow Metab. 28(11):1761-70.
9. Okhuma et al. 2000: Impact of cerebral microcirculatory changes on cerebral blood flow during cerebral vasospasm after aneurysmal subarachnoid hemorrhage. Stroke 31(7):1621-7.
10. Pluta et al. 2009: Cerebral vasospasm following subarachnoid hemorrhage: Time for a new world of thought. Neurol. Res. 31 (2):151-8.
11. Weyer et al. 2006: Evidence-based cerebral vasospasm management. Neurosurg. Focus 21:E8.
12. Rabinstein et al. 2004: Predictors of outcome after endovascular treatment of cerebral vasospasm. Am. J. Neuroradiol. 25(10):1778-82.
13. Romano et al. 2008: Microemboli in aneurysmal subarachnoid hemorrhage. J. Neuroimaging 18(4): 396-401.
14. Macdonald 2008: An endothelin receptor antagonist for treatment of vasospasms after subarachnoid hemorrhage. Expert Opin. Investig. Drugs 17:1761-7.
15. Barth et al. 2007: Effect of nicardipine prolonged-release implants on cerebral vasospasm and clinical outcome after severe aneurysmal subarachnoid hemorrhage: a prospective, randomized, double-blind phase IIa study. Stroke 38(2):330-6.
16. Faraci and Brian 1994: Nitric oxide and the cerebral circulation. Stroke 25(3):692-703.
17. ladecola et al. 1994: Nitric oxide synthase inhibition and cerebrovascular regulation. J. Cereb. Blood Flow Metab. 14(2):175-92.
18. Moncada et al. 1991: Nitric oxide: physiology, pathophysiology, and pharmacology. Pharmacol. Rev. 43(2):109-42.
19. Busse and Mulsch 1990: Induction of nitric oxide synthase by cytokines in vascular smooth muscle cells. FEBS letters 275:87-90.
20. Alonso et al. 1992: Predominant role for nitric oxide in the relaxation induced by acetylcholine in cat cerebral arteries. J. Pharmacol. Exp. Ther. 261:12-20.
21. Faraci 1991: Role of endothelium-derived relaxing factor in cerebral circulation: Large arteries vs. microcirculation. Am. J. Physiol. 261:H1038-42.
22. Busse et al. 1985: The role of endothelium in the control of vascular tone. Basic Res. Cardiol. 80:475-90.
23. Katusic and Sheperd 1991: Endothelium-derived vasoactive factors: li. Endotehlium-dependent concentration. Hypertension 18:11186-92.
24. Hino et al. 1996: Changes in endothelial nitric oxide synthase mRNA during vasospasm after subarachnoid hemorrhage in monkeys. Neurosurgery 39(3):562-7.
25. Galle et al. 1993: Arterial size determines the enhancement of contractile responses after suppression of endothelium-derived relaxing factor formation. Pflugers Arch. 422:564-9.
26. Wellman et al. 2004: Nitric oxide and reactive oxygen species exert opposing effects on the stability of hypoxia-inducible factor-1 alpha (hif-1 alpha) in explants of human pial arteries. FASEB J. 18:379-81.
27. Harder et al. 1987: Possible cellular mechanism for cerebral vasospasm after experimental subarachnoid hemorrhage in the dog. J. Clin. Invest. 80:875-80.
28. Zuccarello et al. 1996: Prevention of subarachnoid hemorrhage-induced cerebral vasospasm by oral administration of endothelin receptor antagonists. J. Neurosurg. 84:503-7.
29. Andaluz et al. 2002: Indications for endovascular therapy for refractory vasospasm after aneurysmal subarachnoid hemorrhage: Experience at the university of Cincinnati. Surg. Neurol. 58:131-8.
30. Aiello et al. 1996: Protein kinase c inhibits delayed rectifier k+ current in rabbit vascular smooth muscle cells. Am. J. Physiol. 271:H109-19.
31. Bonev et al. 1997: Activators of protein kinase c decrease ca2+ spark frequency in smooth muscle cells from cerebral arteries. Am. J. Physiol. 273:C2090-5.
32. Ishiguro et al. 2008: Cellular basis of vasospasm: Role of small diameter arteries and voltage-dependent ca2+ channels. Acta Neurochir. Suppl. 104:95-8.
33. Schini et al. 1994: Inducible nitric oxide synthase in vascular smooth muscle. Arzneimittelforschung 44:432-5.
34. Guo et al. 1995: Mechanism of vascular preservation by a novel NO donor following rat caroti artery intimal injury. Am. J. Physiol. 269:H1122-31.
35. Scott-Burden et al. 1992. Platelet-derived growth factor suppresses and fibroblast growth factor enhances cytokine-induced production of nitric oxide by cultured smooth muscle cells. Effects on cell proliferation. Circ. Res. 71 (5):1088-1100.
36. Scott-Burden and Vanhoutte 1994: Regulation of smooth muscle cell growth by endothelium-derived factors. Tex. Heart inst. J. 21:91-7.
37. Schwartz et al. 1995: The intima: Soil for atherosclerosis and restenosis. Circ. Res. 77:445-65
38. Joly et al. 1992: Balloon injury and interleukin-1 beta induce nitric oxide synthase activity in rat carotid arteries. Circ. Res. 71:331-8.
39. Garg et al. 1989: Nitric-oxide generating vasodilators and 8-bromo-cyclic guanosine monophosphate inhibit mitogenesis and proliferation of cultured rat vascular smooth muscle cells. J. Clin. Invest. 83:1774-7.
40. McNamara et al. 1993: L-arginine inhibits balloon catheter-induced intimal hyperplasia. Biochem. Biophys. Res. Commun. 193:291-6.
41. von der Leyen et al. 1995: Gene therapy inhibiting neointimal vascular lesion: In vivo transfer of endothelial cell nitric oxide synthase gene. PNAS 92:1137-41.
42. Bassenge et al. 1989: Inhibition of thrombocyte aggregation and adhesion by endothelium-derived relaxant factor (edrf) and their pathophysiologic significance. Z. Kardiol. 78 Suppl. 6:54-8.
43. Cahill et al. 2006: Cahill PA, Redmond EM, Foster C, Sitzmann JV. Nitric oxide regulates angiotensin II receptors in vascular smooth muscle cells. Eur J Pharmacol. 1995 Jan 16;288(2):219-29.
44. Bohme 1990: Pharmacology of molsidomine and its active metabolites. Med. Klin. (Munich) 85 Suppl 1:7-10.
45. Rubanyi 1991: Endothelium-derived relaxing and contracting factors. J. Cell Biochem. 46:27-36.
46. Macdonald and Weir 1991: A review of hemoglobin and the pathogenesis of cerebral vasospasm. Stroke 22:971-82.
47. Cook 1995. Mechanisms of cerebral vasospasm in subarachnoid haemorrhage. Pharmacol. Ther. 66:259-84.
48. Seifert et al. 1995: Endothelin concentrations in patients with aneurysmal subarachnoid hemorrhage. Correlation with cerebral vasospasm, delayed ischemic neurological deficits, and volume of hematoma. J. Neurosurg. 82:55-62.
49. Vajkoczy et al. 2000: Intrathecal sodium nitroprusside improves cerebral blood flow and oxygenation in refractory cerebral vasospasm and ischemia in humans. Stroke 31:1195-7.
50. Raabe et al. 2002: Effect of intraventricular sodium nitroprusside on cerebral hemodynamics and oxygenation in poor-grade aneurysm patients with severe, medically refractory vasospasm. Neurosurgery 50:1006-13.
51. Hirsh 1980: Intra-arterial nitroprusside treatment of acute experimental vasospasm. Stroke 11:601-5.
52. Wolf et al. 1998: Reversal of cerebral vasospasm using an intrathecally administered nitric oxide donor. J. Neurosurg. 89:279-88.
53. Egemen N, Türker RK, Sanlidilek U, Zorlutuna A, Bilgiç S, Baskaya M, Unlü A, Caglar S, Spetzler RF, McCormick JM. The effect of intrathecal sodium nitroprusside on severe chronic vasospasm. Neurol Res. 1993 Oct;15(5):310-5.
54. Thomas et al. 1997: Rapid reversal of endothelin-1-induced cerebral vasoconstriction by intrathecal administration of nitric oxide donors. Neurosurgery 40:1245-9.
55. Heros et al. 1976: Reversal of experimental cerebral vasospasm by intravenous nitroprusside therapy. Surg. Neurol. 6:227-9.
56. Goksel et al. 2001: The therapeutic effect of continuous intracisternal I-arginine infusion on experimental cerebral vasospasm. Acta Neurochir. (Wien) 143:277-85.
57. Casthely et al. 1981: Cerebrospinal fluid cyanide after nitroprusside infusion in man. Can. Anaesth. Soc. J. 28:228-31.
58. Hecker et al. 1995 Elevation of circulating no: Its effects on hemodynamics and vascular smooth muscle cell proliferation in rats. Agents Actions Suppl. 45:169-76.
59. Rosenkranz et al. 1996: Clinical pharmacokinetics of molsidomine. Clin. Pharmacokinet. 30:372-84.
60. Serruys et al. 1987: Long-acting coronary vasodilatory action of the molsidomine metabolite sin 1: A quantitative angiographic study. Eur. Heart J. 8:263-70.
61. Loch Macdonald 2006: Management of cerebral vasospasm. Neurosurg. Rev. 29:179-93.
62. Pluta 2005: Delayed cerebral vasospasm and nitric oxide: Review, new hypothesis, and proposed treatment. Pharmacol. Ther. 105:23-56.
63. Kramer and Fletcher 2009: Do endothelin-receptor antagonists prevent delayed neurological deficits and poor outcomes after aneurysmal subarachnoid hemorrhage?: A meta-analysis. Stroke 40:3403-6.
64. van den Bergh et al. 2004: Potentials of magnesium treatment in subarachnoid haemorrhage. Magnes. Res. 17:301-13.
65. Komotar et al. 2007: Advances in vasospasm treatment and prevention. J. Neurol. Sci. 261:134-42.
66. Rinkel and Klijn 2009: Prevention and treatment of medical and neurological complications in patients with aneurysmal subarachnoid haemorrhage. Pract. Neurol. 9:195-209.
67. Smith et al. 2002: Nitric oxide gas decreases endothelin-1 mRNA in cultured pulmonary artery endothelial cells. Nitric Oxide 6(2):153-9.
68. Wagner et al. 2003: Modulation of circulating endothelin-1 and big endothelin by nitric oxide inhalation following left ventricular assist device implantation. Circulation 108 Suppl. 1:II278-84.
69. ASANO Shimokawa H, Seto M, Katsumata N, Amano M, Kozai T, Yamawaki T, Kuwata K, Kandabashi T, Egashira K, Ikegaki I, Asano T, Kaibuchi K, Takeshita A.Rho-kinase-mediated pathway induces enhanced myosin light chain phosphorylations in a swine model of coronary artery spasm.Cardiovasc Res. 1999 Sep;43(4):1029-39.
70. Chitaley K, Webb RC. Nitric oxide induces dilation of rat aorta via inhibition of rho-kinase signalling.Hypertension. 2002 Feb;39(2 Pt 2):438-42.
71. Keyrouz and Diringer 2007: Clinical review: Prevention and therapy of vasospasm in subarachnoid hemorrhage. Critical Care 11 (4):220.
72. de Rooij et al. 2007: Incidence of subarachnoid haemorrhage: A systematic review with emphasis on region, age, gender and time trends. Journal of Neurology, Neurosurgery, and Psychiatry 78 (12): 1365-72.
73. Bassenge E, Schneider HT, Daiber A. [Oxidative stress and cardiovascular diseases] Dtsch Med Wochenschr. 2005 Dec 16;130(50):2904-9. [Article in German]
81. Pluta RM, Rak R, Wink DA, Woodward JJ, Khaldi A, Oldfield EH, Watson JC. Effects of nitric oxide on reactive oxygen species production and infarction size after brain reperfusion injury. Neurosurgery. 2001 Apr;48(4):884-92; discussion 892-3.
82. Pluta RM, Thompson BG, Afshar JK, Boock RJ, Iuliano B, Oldfield EH. Nitric oxide and vasospasm. Acta Neurochir Suppl. 2001 ;77:67-72.
   Macdonald RL, Kassell NF, Mayer S, Ruefenacht D, Schmiedek P, Weidauer S, Frey A, Roux S, Pasqualin A; CONSCIOUS-1 Investigators.Stroke. 2008 Nov;39(11):3015-21. Epub 2008 Aug 7Clazosentan to overcome neurological ischemia and infarction occurring after subarachnoid hemorrhage (CONSCIOUS-1): randomized, double-blind, placebo-controlled phase 2 dose-finding trial.
84. Pearl JD, Macdonald RL. Vasospasm after aneurysmal subarachnoid hemorrhage: need for further study. Acta Neurochir Suppl2008;105:207-10.
85. Sobey CG, Faraci FM. Subarachnoid haemorrhage: what happens to the cerebral arteries? Clin Exp Pharmacol Physiol. 1998 Nov;25(11):867-76.
86.Dirnagl U, Becker K, Meisel A. Preconditioning and tolerance against cerebral ischaemia: from experimental strategies to clinical use.Lancet Neurol. 2009 Apr;8(4):398-412.
87. Xing B, Chen H, Zhang M, Zhao D, Jiang R, Liu X, Zhang S. Ischemic postconditioning inhibits apoptosis after focal cerebral ischemia/reperfusion injury in the rat.Stroke.2008 Aug;39(8):2362-9. Epub 2008 Jun 26.
88. Zhao L, Nowak TS Jr. CBF changes associated with focal ischemic preconditioning in the spontaneously hypertensive rat.J Cereb Blood Flow Metab. 2006 Sep;26(9):1128-40. Epub 2006 Jan 11.
89. Kawahara N, Ruetzler CA, Klatzo I. Protective effect of spreading depression against neuronal damage following cardiac arrest cerebral ischaemia.Neurol Res.1995 Feb;17(1):9-16.
90. Kawahara N, Ruetzler CA, Mies G, Klatzo I. Cortical spreading depression increases protein synthesis and upregulates basic fibroblast growth factor.Exp Neurol.1999 Jul;158(1):27-36.
91. Taga K, Patel PM, Drummond JC, Cole DJ, Kelly PJ. Transient neuronal depolarization induces tolerance to subsequent forebrain ischemia in rats.Anesthesiology.1997 Oct;87(4):918-25.
92. Douen AG, Akiyama K, Hogan MJ, Wang F, Dong L, Chow AK, Hakim A. Preconditioning with cortical spreading depression decreases intraischemic cerebral glutamate levels and down-regulates excitatory amino acid transporters EAAT1 and EAAT2 from rat cerebal cortex plasma membranes.J Neurochem.2000 Aug;75(2):812-8.
93. Lai AY, Todd KG. Microglia in cerebral ischemia: molecular actions and interactions.Can J Physiol Pharmacol.2006 Jan;84(1):49-59.
94. Mabuchi T, Kitagawa K, Ohtsuki T, Kuwabara K, Yagita Y, Yanagihara T, Hori M, Matsumoto M. Contribution of microglia/macrophages to expansion of infarction and response of oligodendrocytes after focal cerebral ischemia in rats.2000 Jul;31(7):1735-43.

## Claims

1. A nitric oxide (NO) donor for use in the treatment, prevention and/or amelioration of nitric oxide deficiency induced disturbances of the cerebral microcirculation of a human subject.

2. The NO donor of claim 1, which is a compound having the formula I 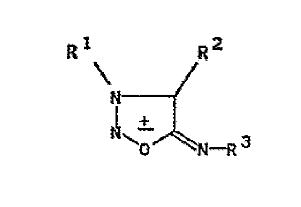 wherein
R¹is morpholine, N-heterocyclyl or N(R⁴)₂;
R² is H, alkyl, halogen, alkenyl, alkynyl, aryl, aralkyl, aralkenyl, alkylene, alkenylene, cycloalkyl, cycloalkylene or N-heterocyclyl;
R³ is H, -NO, -SO₂R⁴, -C(O)OR⁴, -C(O)R⁴, -CH₂NHC(O)R⁴, -CHR⁴, -NR⁴, - CHR⁴OC(O)R⁴ and -C(O)CHR⁴N⁺H₂; and
each R⁴ is independently hydrogen, alkyl, alkenyl, alkynyl, aryl, aralkyl or aralkenyl;
or pharmaceutically acceptable salts thereof.

3. The NO donor of claim 2, wherein R¹ is morpholine, R² is H and R³ is -C(O)OC₂H₅.

4. The NO donor of claim 2 or 3 which is N-ethoxycarbonyl-3-morpholinosydnonimine (Molsidomine).

5. The NO donor of claim 4 which has the formula II

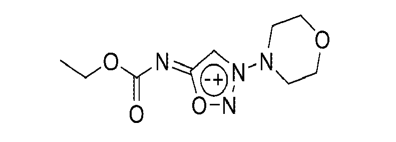

6. The NO donor of claim 2, wherein R¹ is morpholine, R² is H and R³ is H.

7. The NO donor of claim 5 which is 3-morpholinosydnonimine (SIN-1).

8. The NO donor of claim 7 which has the formula III

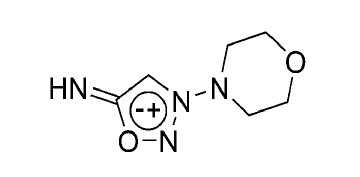

9. The NO donor of claim 3 which is 3-(3,3-dimethyl-1-oxo-1,4-thiazine-4-yl)sydnonimine, 3-(3,3-dimethyl-1,1-dioxo-1,4-thiazine-4-yl)sydnonimine, 3-(3,3-dimethyl-1,4-thiazine-4-yl)sydnonimine, 3-(cis-2,6-dimethylpiperidino)sydnonimine, 3-morpholinosydnoniminechloride (Linsidomine; SIN-1) or 3-(cis-2,6-dimethylpiperidino)-N-(4-methoxybenzoyl)sydnonimine (Pirsidomin) or N-ethoxycarbonyl-3-morpholinosydnonimine (Molsidomine).

10. The NO donor of any one of the preceding claims, wherein the disturbances of the cerbral microcirculation and/or macrocirculation cause cerebrovascular spasms (CVS) and/or malperfusion of brain parenchyma caused by blood vessel and/or blood flow dysregulation.

11. The NO donor of any one of the preceding claims, wherein the NO deficiency induced disturbances are due to an intracranial hemorrhage or stroke.

12. The NO donor of any one of the preceding claims, wherein the intracranial hemorrhage results from a traumatic or a non-traumatic cause.

13. The NO donor of any one of the preceding claims, wherein intracranial hemorrhage is an intra-axial hemorrhage (cerebral hemorrhage) or extra-axial hemorrhage.

14. The NO donor of any one of the preceding claims, wherein the extra-axial hemorrhage is an epidural, subdural or subarachnoid hemorrhage.

15. The NO donor of any one of the preceding claims, wherein said NO donor is administered at a dose of more than about 2 mg per hour.
